# EUROPEAN PATENT APPLICATION

(11) **EP 3 936 126 A1**
(43) Date of publication of application: **12.01.2022**
(21) Application number: 20766360.0
(22) Date of filing: 06.03.2020
(51) Int. Cl.: A61K 31/4045, A61P 35/00, C07D 403/06

(54) **COMBINATION OF IAP INHIBITOR AND IMMUNE CHECKPOINT INHIBITOR**

(30) Priority: 07.03.2019 CN 201910172867
(71) Applicant: CHIA TAI TIANQING PHARMACEUTICAL GROUP CO., LTD., Lianyungang, Jiangsu 222062 (CN)
(72) Inventor: LIU, Yingchun, Nanjing, Jiangsu 210032 (CN); XU, Zhaobing, Nanjing, Jiangsu 210032 (CN); HAN, Shuhua, Nanjing, Jiangsu 210032 (CN); HU, Lihong, Nanjing, Jiangsu 210032 (CN); DING, Charles Z., Nanjing, Jiangsu 210032 (CN); XIA, Yuanfeng, Nanjing, Jiangsu 210032 (CN); TONG, Haijun, Nanjing, Jiangsu 210032 (CN); HE, Lihua, Lianyungang, Jiangsu 222062 (CN); TIAN, Xin, Lianyungang, Jiangsu 222062 (CN)
(74) Representative: Grünecker Patent- und Rechtsanwälte PartG mbB
(86) International application number: PCT/CN2020/078234
(87) International publication number: WO 2020/177765

(57) **Abstract**

The present invention relates to a combination of a compound represented by formula (I), an isomer thereof or a pharmaceutically acceptable salt thereof which are used as an IAP inhibitor, and an immune checkpoint inhibitor; and use of the combination in the preparation of a cancer treatment drug.

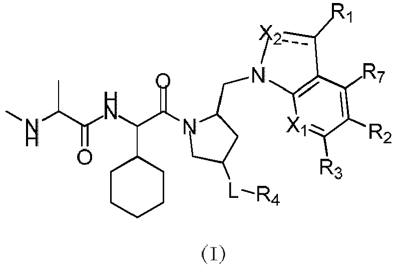

## Description

### CROSS-REFERENCE TO RELATED APPLICATION

The present disclosure claims the priority and benefit of Chinese Patent Application No. 201910172867.8 filed with the National Intellectual Property Administration, PRC on March 7, 2019, the disclosed contents of which are incorporated herein by reference in their entirety.

### TECHNICAL FIELD

The present disclosure belongs to the field of biological medicines, and relates to a combination of a compound represented by formula (I), an isomer thereof or a pharmaceutically acceptable salt thereof which is used as an IAP inhibitor, and an immune checkpoint inhibitor; and use of the combination in the preparation of a cancer treatment drug.

### BACKGROUND

The evasion of apoptosis and the escape of immune check are two important pathways for the occurrence and development of tumor. Members of the inhibitor of apoptosis proteins (IAPs) gene family play a role in both pathways through which cancer occurs. Firstly, IAPs, as inhibitor of apoptosis proteins, have their own role in inhibiting the cell apoptosis. IAP inhibitors can inhibit such an anti-apoptotic effect, which in turn acts to promote the apoptosis in tumor cells. Mechanistically, IAP inhibitors bind to the N-terminus of cIAPs, inducing self-ubiquitination and subsequent proteasome-mediated degradation of cIAPs. Thus, tumor cells are rendered more sensitive to TNF-α (tumor necrosis factor a)-mediated apoptosis. Secondly, IAP inhibitors have also been found to have certain immunomodulatory effects. Mechanistically, IAP inhibitors can promote the proliferation of CD4+ T cells and CD8+ T cells, as well as the viability of these cells. Meanwhile, they can also promote T cells and NK cells to secrete IL-2, IFN-γ and other cytokines that enhance the tumor immunity.

At present, many drug molecules have entered the clinical research, such as LCL-161, Debio 1143, BI-891065, and ASTX-660. In order to obtain more effective therapeutic agents for clinical use, it can be attempted to administer immune checkpoint inhibitors in combination with the immunomodulatory function of IAP inhibitors to achieve the ultimate goal of treating tumors.

### SUMMARY

In one aspect, the present disclosure provides a combination comprising a compound represented by formula (I) or a pharmaceutically acceptable salt thereof, and an immune checkpoint inhibitor.

In another aspect, the present disclosure provides a combined pharmaceutical composition comprising the combination of the present disclosure, and a pharmaceutically acceptable excipient. In another aspect, the present disclosure provides a kit comprising the combined pharmaceutical composition of the present disclosure and instructions for use of a compound represented by formula (I) or a pharmaceutically acceptable salt thereof in combination with an immune checkpoint inhibitor in the treatment of cancer.

In another aspect, the present disclosure provides use of the combination, the combined pharmaceutical composition or the kit of the present disclosure in the preparation of a cancer treatment drug. The present disclosure also provides a method for treating cancer, comprising administering to a subject an effective amount of the combination, the combined pharmaceutical composition or the kit of the present disclosure. The present disclosure also provides use of the combination, the combined pharmaceutical composition or the kit of the present disclosure in the treatment of cancer.

In another aspect, the present disclosure provides use of a compound represented by formula (I) or a pharmaceutically acceptable salt thereof in combination with an immune checkpoint inhibitor in the preparation of a cancer treatment drug. The present disclosure also provides a method for treating cancer, comprising administering to a subject an effective amount of a compound represented by formula (I) or a pharmaceutically acceptable salt thereof and an immune checkpoint inhibitor. The present disclosure also provides use of a compound represented by formula (I) or a pharmaceutically acceptable salt thereof in combination with an immune checkpoint inhibitor in the treatment of cancer.

### Compound Represented by Formula (I)

As used herein, the compound represented by formula (I) of the present disclosure, an isomer thereof or a pharmaceutically acceptable salt thereof is as follows: 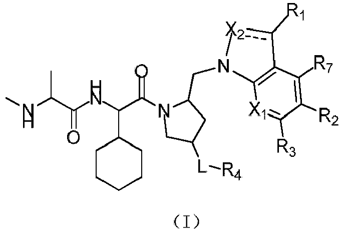 wherein,
X₁ is selected from C(R₅) and N;
X₂ is selected from C(R₆), N, O, and S;
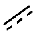 is selected from a single bond and a double bond;
L is selected from a single bond and -O-;
R₁ is selected from -C(=O)NH₂, CN, C₁₋₅ alkyl, C₁₋₅ heteroalkyl, phenyl, 5- to 6-membered heteroaryl, and 5- to 6-membered heterocycloalkyl; the C₁₋₅ alkyl, C₁₋₅ heteroalkyl, phenyl, 5-to 6-membered heteroaryl and 5- to 6-membered heterocycloalkyl are optionally substituted with 1, 2 or 3 R;
R₂ is selected from H, halogen, CN, COOH, -C(=O)NH₂, C₁₋₄ alkyl, and C₁₋₄ heteroalkyl; the C₁₋₄ alkyl and C₁₋₄ heteroalkyl are optionally substituted with 1, 2 or 3 R;
R₃ and R₇ are each independently selected from H, halogen and C₁₋₄ alkyl; the C₁₋₄ alkyl is optionally substituted with 1, 2 or 3 R;
R₄ is selected from H, phenyl, and 5- to 6-membered heteroaryl;
R₅ is selected from H and halogen;
R₆ is selected from H, halogen, C₁₋₄ alkyl, C₁₋₄ heteroalkyl, CN, and COOH; the C₁₋₄ alkyl and C₁₋₄ heteroalkyl are optionally substituted with 1, 2 or 3 R;
R is selected from halogen, OH, CN, CH₃, CH₃CH₂, CH₃CH₂CH₂, CH(CH₃)₂, OCH₃, OCF₃, CHF₂, CH₂F, and NH₂; and
the C₁₋₄ heteroalkyl, C₁₋₅ heteroalkyl, 5- to 6-membered heterocycloalkyl and 5- to 6-membered heteroaryl each contain 1, 2 or 3 heteroatoms or heteroatom radicals independently selected from -NH-, -O-, -S-, N, -C(=O)O-, -C(=O)-, -C(=O)NH-, -C(=S)-, -S(=O)-, -S(=O)₂-, -C(=NH)-, -S(=O)₂NH-, -S(=O)NH-, and -NHC(=O)NH-.

In some embodiments of the present disclosure, the above compound represented by formula (I), an isomer thereof or a pharmaceutically acceptable salt thereof is selected from 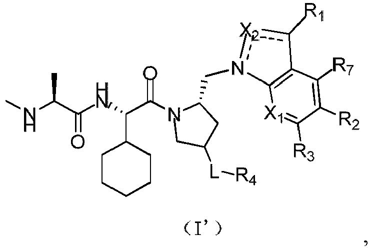 wherein 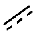 , X₁, X₂, L, R₁, R₂, R₃, R₄ and R₇ are as defined herein.

In some embodiments of the present disclosure, the above X₂ is selected from C(R₆) and N.

In some embodiments of the present disclosure, the above X₂ is selected from C(H), C(Cl), C(CH₃), and N.

In some embodiments of the present disclosure, the above R₁ is selected from -C(=O)NH₂, CN, CH₃, CH₃CH₂, C₁₋₅ alkyl-C(=O)-, C₁₋₄ alkyl-C(=O)-, C₁₋₅ alkyl-S(=O)₂-, C₁₋₅ alkyl-N(H)C(=O)-, C₁₋₄ alkyl-N(H)C(=O)-, (C₁₋₂ alkyl)₂-N-C(=O)-, phenyl, 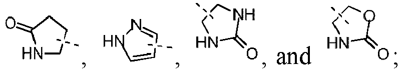 the CH₃, CH₃CH₂, C₁₋₅ alkyl-C(=O)-, C₁₋₄ alkyl-C(=O)-, C₁₋₅ alkyl-S(=O)₂-, C₁₋₅ alkyl-N(H)C(=O)-, C₁₋₄ alkyl-N(H)C(=O)-, (C₁₋₂ alkyl)₂-N-C(=O)-, phenyl, 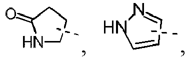 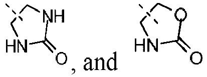 are optionally substituted with 1, 2 or 3 R.

In some embodiments of the present disclosure, the above R₁ is selected from

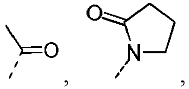

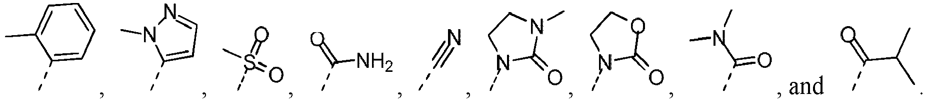

In some embodiments of the present disclosure, the above R₂ is selected from H, halogen, C₁₋₄ alkyl, and C₁₋₄ alkyl-O-; the C₁₋₄ alkyl and C₁₋₄ alkyl-O- are optionally substituted with 1, 2 or 3 halogens.

In some embodiments of the present disclosure, the above R₂ is selected from H, F, Cl, Br, CF₃, and OCF₃. In some embodiments of the present disclosure, the above R₃ and R₇ are each independently selected from H, F and Cl.

In some embodiments of the present disclosure, the above R₄ is selected from H and

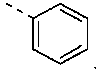

In some embodiments of the present disclosure, the above R₅ is selected from H and Cl.

In some embodiments of the present disclosure, the above R₆ is selected from H, Cl and CH₃.

In some embodiments of the present disclosure, the above structural unit 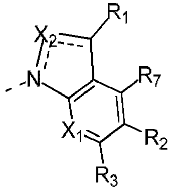 is selected from

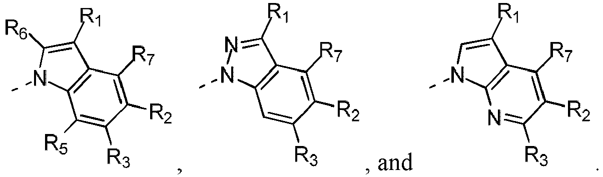

In some embodiments of the present disclosure, the above structural unit 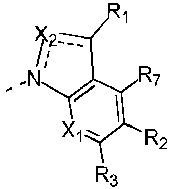 is selected from 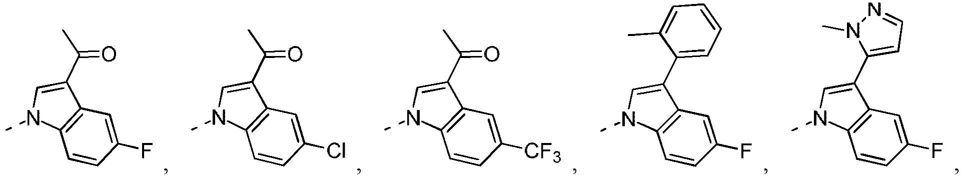 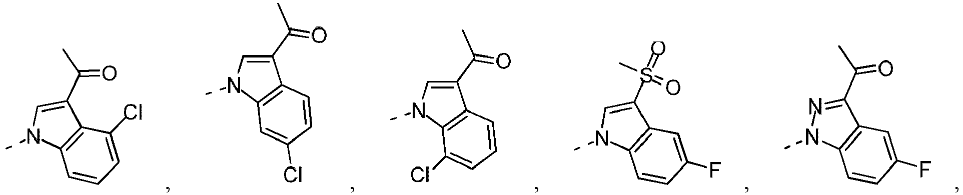 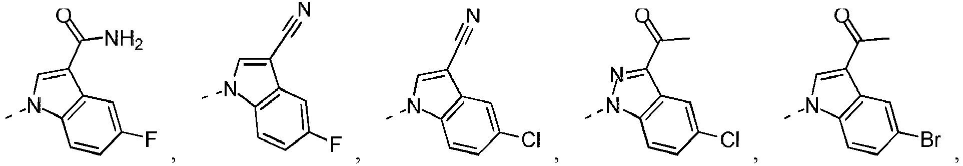 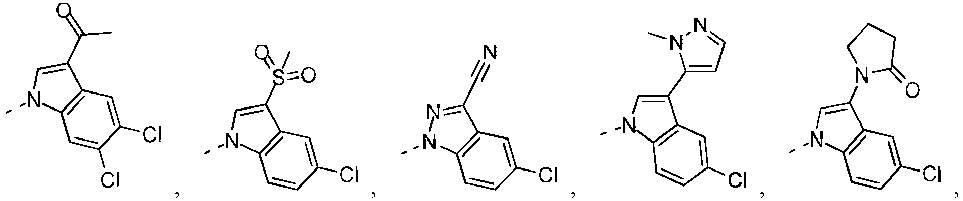 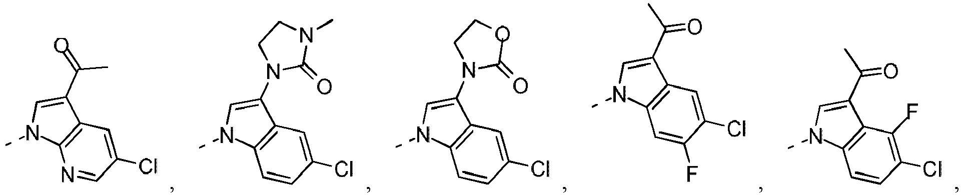 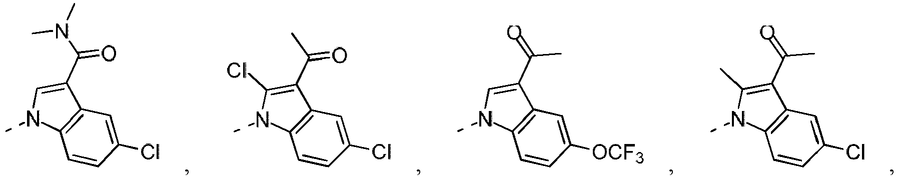 and

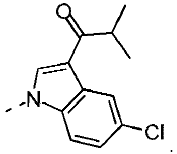

In some embodiments of the present disclosure, the above compound, an isomer thereof or a pharmaceutically acceptable salt thereof is selected from 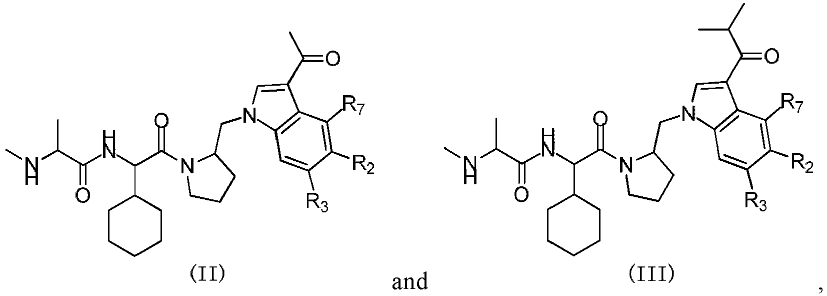 wherein R₂, R₃ and R₇ are as defined herein.

In some embodiments of the present disclosure, the above compound, an isomer thereof or a pharmaceutically acceptable salt thereof is selected from 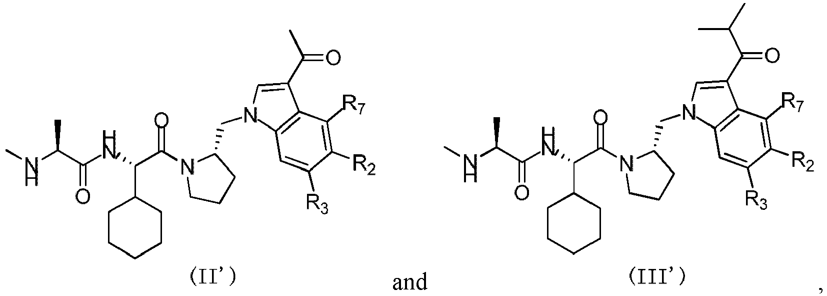 wherein R₂, R₃ and R₇ are as defined herein.

In some embodiments of the present disclosure, the compound represented by formula (I), an isomer thereof or a pharmaceutically acceptable salt thereof is selected from

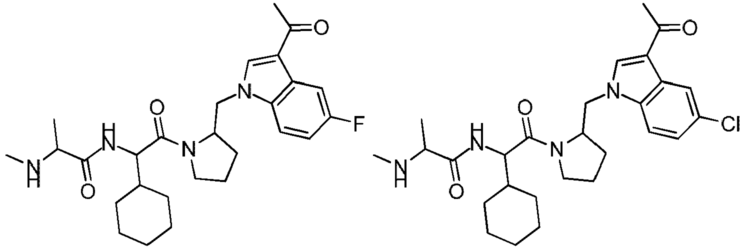

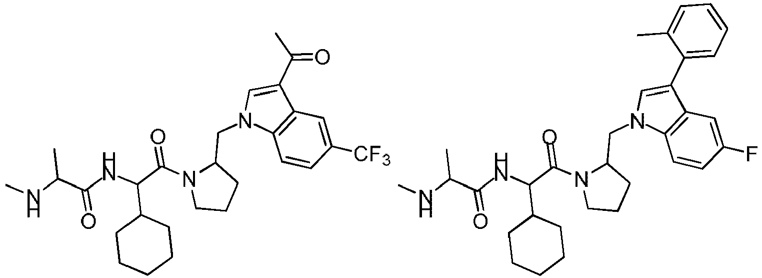

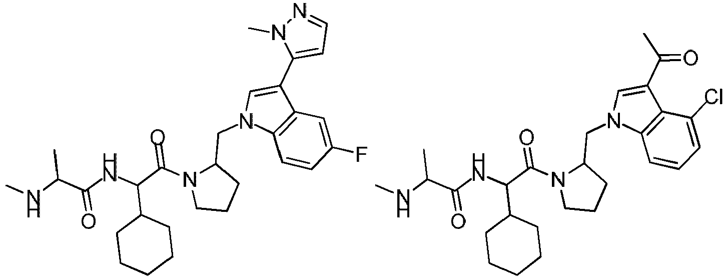

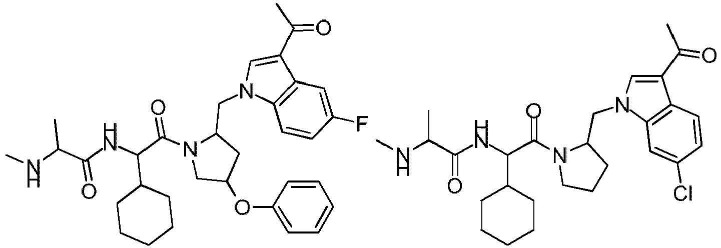

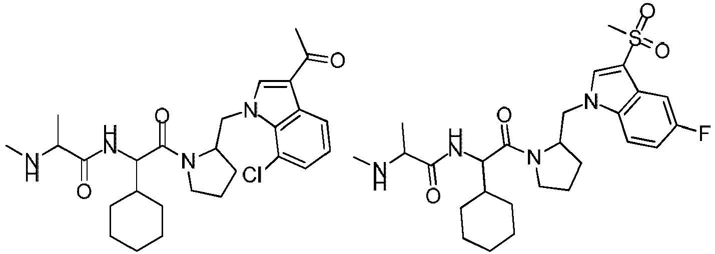

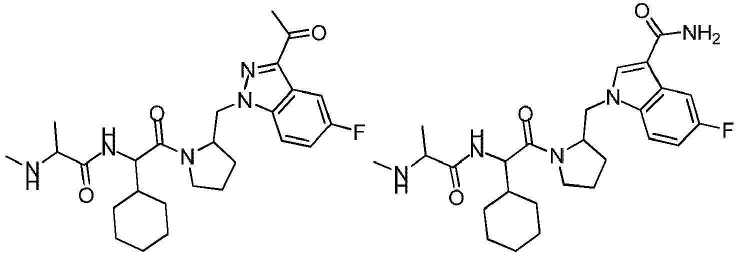

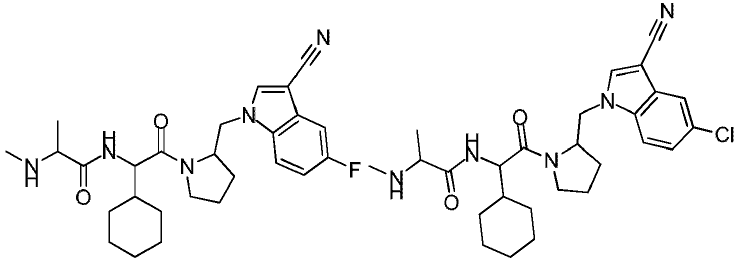

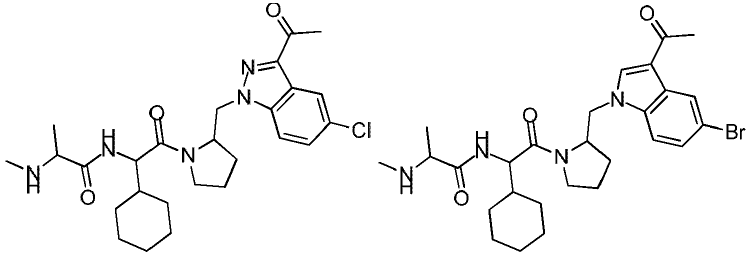

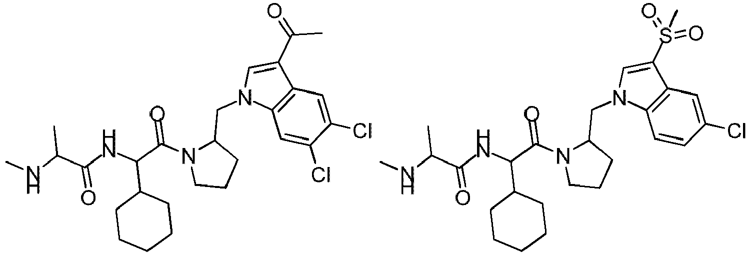

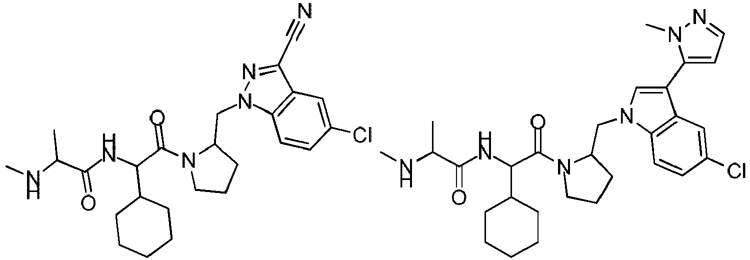

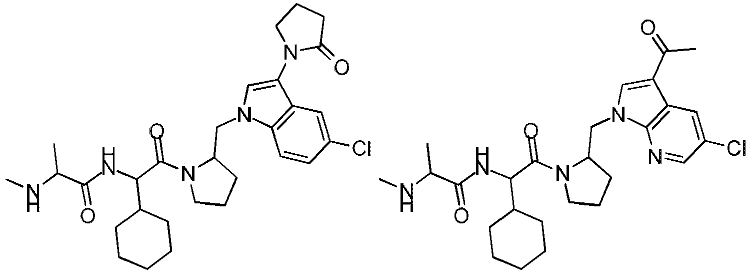

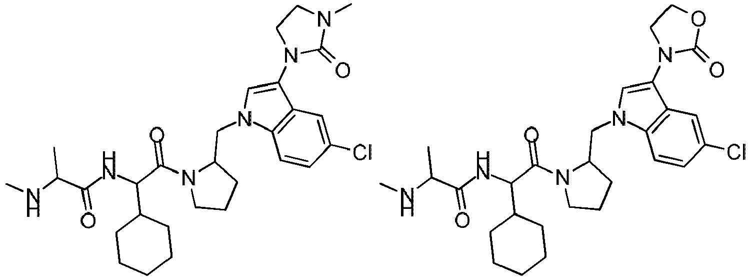

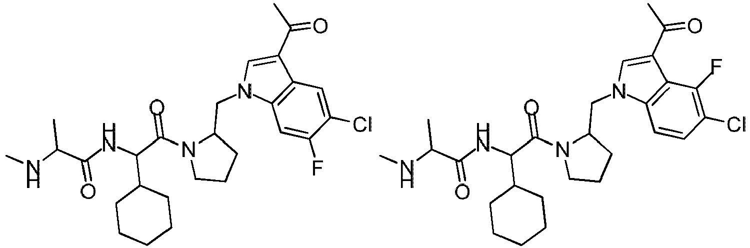

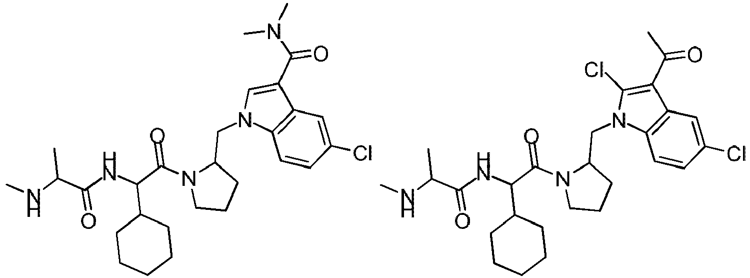

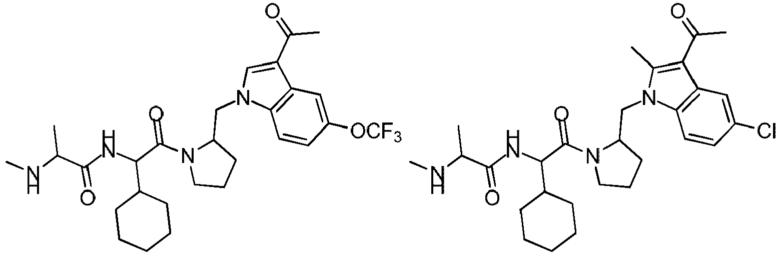

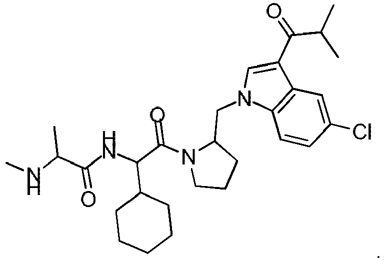

In some embodiments of the present disclosure, the above compound represented by formula (I), an isomer thereof or a pharmaceutically acceptable salt thereof is selected from

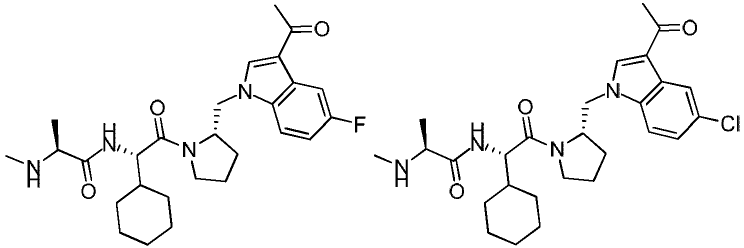

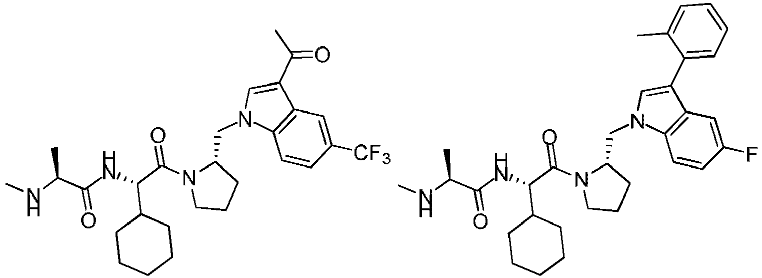

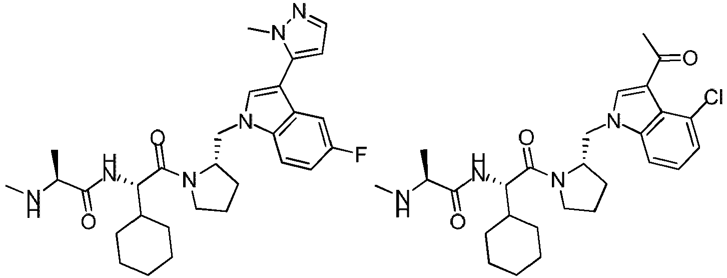

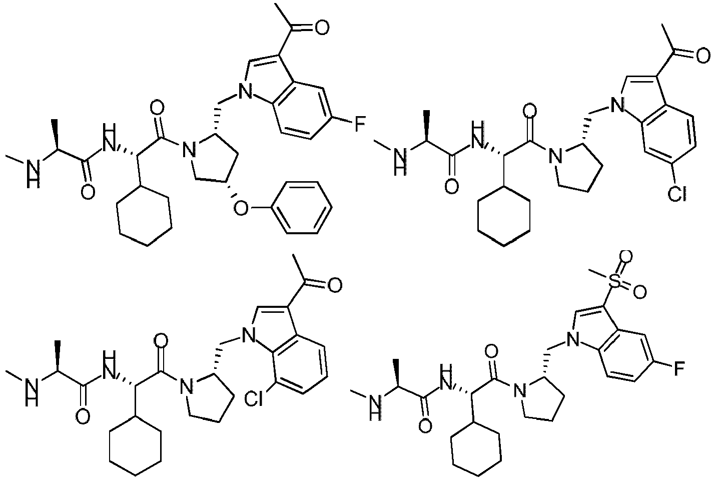

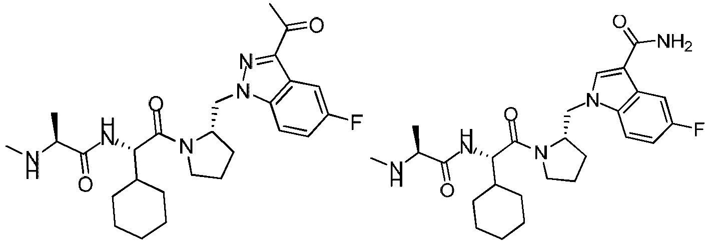

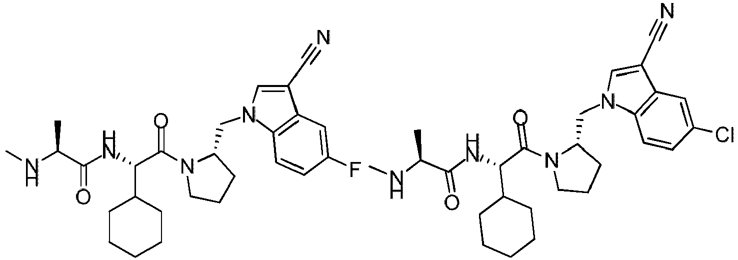

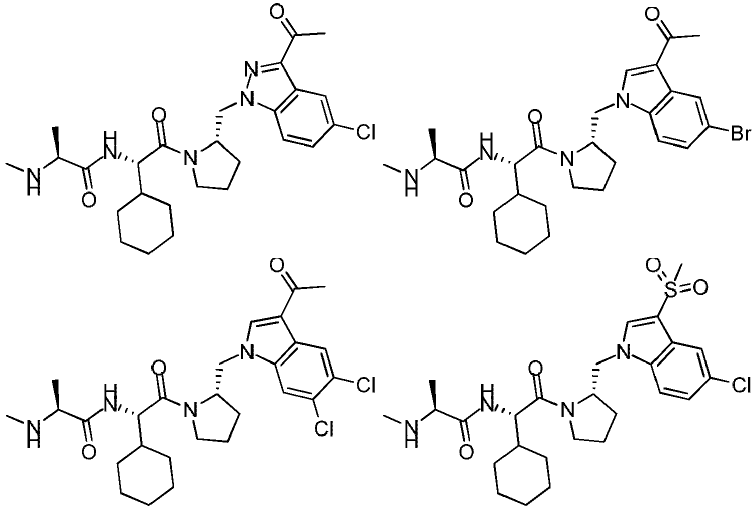

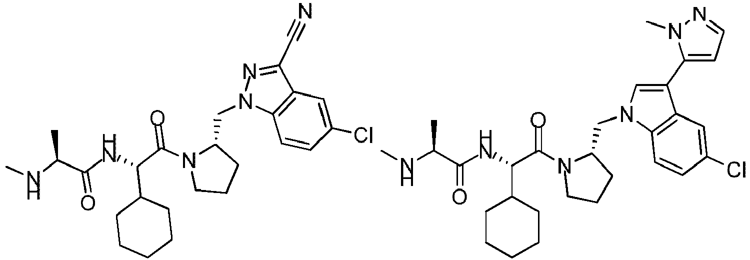

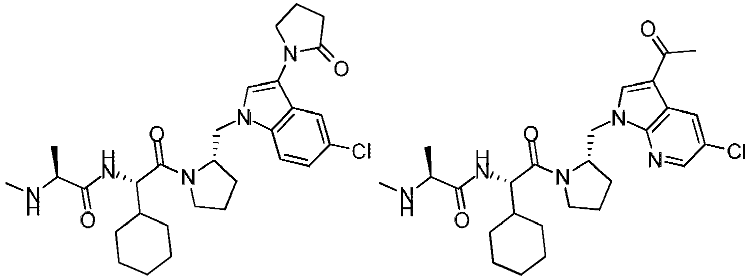

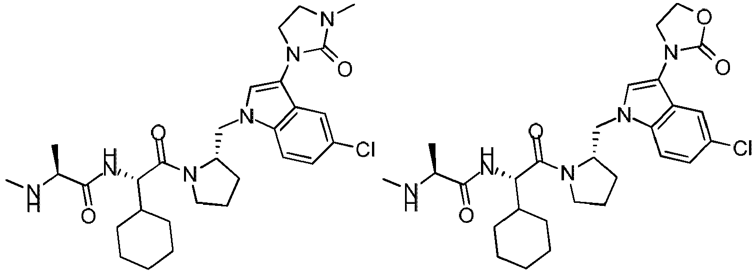

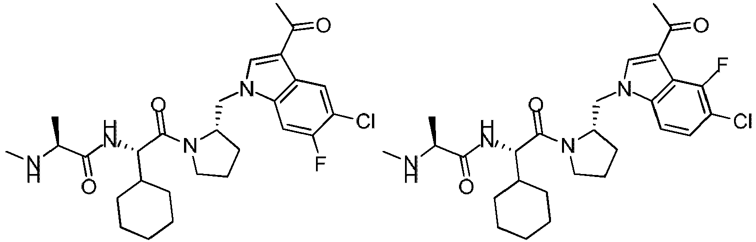

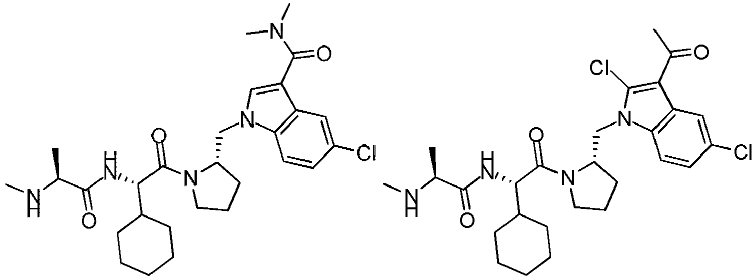

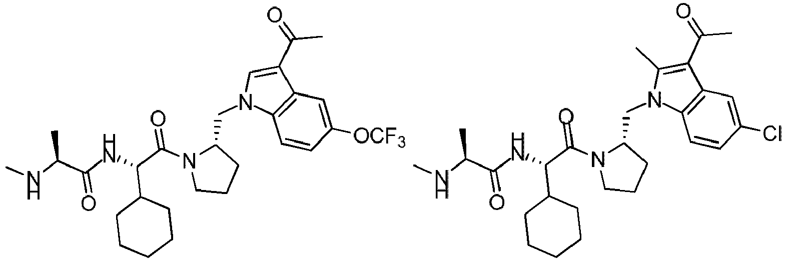

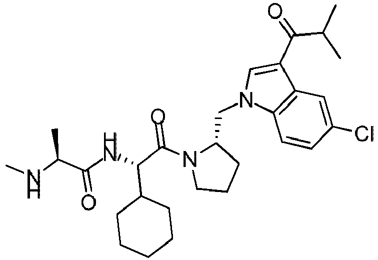

### Immune Checkpoint Inhibitor

As used herein, the immune checkpoint inhibitor is selected from an anti-PD-1 antibody, an anti-PDL1antibody and an anti-CTLA-4 antibody. In some embodiments of the present disclosure, the immune checkpoint inhibitor is selected from an anti-PD-1 antibody. In some embodiments of the present disclosure, the anti-PD-1 antibody is selected from *InVivo*Plus anti-mouse PD-1 (CD279) (batch No. 695318A1B, Bio Cell).

In some embodiments of the present disclosure, the combination is a combination for use in the treatment of cancer.

In some embodiments of the present disclosure, the combined pharmaceutical composition is a pharmaceutical composition for use in the treatment of cancer.

In some embodiments of the present disclosure, the kit is a kit for use in the treatment of cancer.

In some embodiments of the present disclosure, the cancer is selected from a cancer that benefits from the inhibition of IAP. In some embodiments of the present disclosure, the cancer is selected from a cancer that benefits from the inhibition of cIAP1. In some embodiments of the present disclosure, the cancer is selected from breast cancer. In some embodiments of the present disclosure, the cancer is selected from triple-negative breast cancer. In some embodiments of the present disclosure, the cancer is selected from triple-negative breast cancer that benefits from the inhibition of cIAP1.

In some embodiments of the present disclosure, the combined pharmaceutical composition comprises a pharmaceutical composition of a compound represented by formula (I) or a pharmaceutically acceptable salt thereof, and a pharmaceutical composition of an immune checkpoint inhibitor. In some embodiments of the present disclosure, the combined pharmaceutical composition comprises a pharmaceutical composition of a compound represented by formula (I) or a pharmaceutically acceptable salt thereof, and a pharmaceutical composition of an anti-PD-1 antibody.

In some embodiments of the present disclosure, the kit comprises a pharmaceutical composition of a compound represented by formula (I) or a pharmaceutically acceptable salt thereof, a pharmaceutical composition of an immune checkpoint inhibitor, and instructions for use of a compound represented by formula (I) or a pharmaceutically acceptable salt thereof in combination with an immune checkpoint inhibitor in the treatment of cancer. In some embodiments of the present disclosure, the kit comprises a pharmaceutical composition of a compound represented by formula (I) or a pharmaceutically acceptable salt thereof, a pharmaceutical composition of an anti-PD-1 antibody, and instructions for use of a compound represented by formula (I) or a pharmaceutically acceptable salt thereof in combination with an anti-PD-1 antibody in the treatment of cancer.

In some embodiments of the present disclosure, the pharmaceutical composition of the compound represented by formula (I) or a pharmaceutically acceptable salt thereof comprises a compound represented by formula (I) or a pharmaceutically acceptable salt thereof, and a pharmaceutically acceptable excipient. In some embodiments of the present disclosure, the pharmaceutical composition of the compound represented by formula (I) or a pharmaceutically acceptable salt thereof is in the form of a solid formulation, preferably a capsule or a tablet.

In some embodiments of the present disclosure, the pharmaceutical composition of the immune checkpoint inhibitor comprises an immune checkpoint inhibitor and a pharmaceutically acceptable excipient. In some embodiments of the present disclosure, the pharmaceutical composition of the immune checkpoint inhibitor is in the form of a liquid formulation, preferably a water-soluble injection, including but not limited to, a water-soluble formulation without lyophilization or a water-soluble formulation reconstituted from a lyophilized powder.

The combination of the present application may also comprise an additional therapeutic agent. In some embodiments, the additional therapeutic agent may be a cancer therapeutic agent known in the art, preferably a breast cancer therapeutic agent, more preferably a triple-negative breast cancer therapeutic agent.

### Mode of Administration

The contents below are not intended to limit the mode of administration of the combination of the present disclosure.

The components in the combination of the present disclosure can each be formulated separately into a pharmaceutical composition, or some or all of the components can be co-formulated into a pharmaceutical composition. In some embodiments, the combination of the present disclosure can be formulated into a pharmaceutical composition suitable for a single administration or multiple administrations.

The components in the combination of the present disclosure can each be administered separately, or some or all of the components can be co-administered. The components in the combination of the present disclosure can be administered in a substantially asynchronous manner, or some or all of the components are administered in a substantially synchronous manner. The components in the combination of the present disclosure can have the same or different administration periods.

The components in the combination of the present disclosure can each be administered independently in various suitable routes, including but not limited to, oral administration or parenteral administration (intravenous, intramuscular, subcutaneous, intraperitoneal, spinal or other parenteral routes of administration, for example, by injection or infusion). In some embodiments, the components in the combination of the present disclosure can each be administered independently by oral administration or by injection, for example, intravenous injection or intraperitoneal injection.

The components in the composition of the present disclosure can each independently be a suitable dosage form, including but not limited to, tablet, lozenge, pill, capsule (for example, hard capsule, soft capsule, enteric capsule and microcapsule), elixir, granule, syrup, injection (intramuscular, intravenous and intraperitoneal), granule, emulsion, suspension, solution, pulvis and dosage forms of sustained release formulations for oral or non-oral administration.

The components in the combination of the present disclosure can independently contain a pharmaceutically acceptable carrier and/or excipient.

### Definitions and Description

Unless otherwise specified, the following terms and phrases used herein are intended to have the following meanings. A specific term or phrase, if not particularly defined, should not be considered as uncertain or ambiguous, but should be understood as its ordinary meaning. When a trade name appears herein, it is intended to refer to the corresponding commodity thereof or an active ingredient thereof. The term "pharmaceutically acceptable" used herein is intended to refer to those compounds, materials, compositions and/or dosage forms which, within the scope of reliable medical judgment, are suitable for use in contact with the tissues of human beings and animals without excessive toxicity, irritation, allergic response, or other problems or complications, and commensurate with a reasonable benefit/risk ratio.

The term "pharmaceutically acceptable" is used herein for those compounds, materials, compositions, and/or dosage forms which are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of a compound of the present disclosure, which is prepared from a compound having particular substituent(s) as found by the present disclosure and a relatively non-toxic acid or base. When a compound of the present disclosure contains a relatively acidic functional group, a base addition salt may be obtained by contacting the neutral form of such compound with a sufficient amount of a base in a pure solution or a suitable inert solvent. When a compound of the present disclosure contains a relatively basic functional group, an acid addition salt may be obtained by contacting the neutral form of such compound with a sufficient amount of an acid in a pure solution or a suitable inert solvent. Examples of pharmaceutically acceptable acid addition salts include inorganic acid salts. Certain specific compounds of the present disclosure contain basic and acidic functional groups and thus may be converted to any of base addition salts or acid addition salts.

The pharmaceutically acceptable salts of the present disclosure may be synthesized from the parent compounds containing acid radicals or basic radicals by conventional chemical methods. Generally, such salts are prepared by the following method: reacting these compounds in the form of free acid or base with a stoichiometric amount of an appropriate base or acid in water or an organic solvent or a mixture of both.

The compounds of the present disclosure may have specific geometric isomeric form or stereoisomeric form. The present disclosure contemplates all such compounds, including cisand trans-isomers, (-)- and (+)-enantiomers, (*R*)- and (*S*)-enantiomers, diastereomers, (D)-isomers, (L)-isomers, as well as the racemic mixtures and other mixtures thereof, such as enantiomerically or diastereomerically enriched mixtures, and all these mixtures fall within the scope of the present disclosure. Additional asymmetric carbon atom(s) may be present in the substituent(s) such as alkyl. All these isomers and the mixtures thereof are included in the scope of the present disclosure.

Unless otherwise specified, the term "enantiomer" or "optical isomer" refers to either of a pair of stereoisomers that are the mirror images of each other.

Unless otherwise specified, the term "cis-trans isomer" or "geometric isomer" refers to an isomer resulting from the fact that the double bond or the single bond between ring-forming carbon atoms cannot rotate freely.

Unless otherwise specified, the term "diastereomer" refers to either of a pair of stereoisomers, of which the molecule has two or more chiral centers and which has a non-mirror-image relationship with the other stereoisomer of the molecule.

Unless otherwise specified, "(+)" means right-handed, "(-)" means left-handed, and "(±)" means racemic.

Unless otherwise specified, the wedge-shaped solid-line bond ( 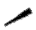 ) and the wedge-shaped dashed-line bond ( 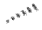 ) are used to represent the absolute configuration of a stereogenic center, and the straight solid-line bond ( 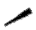 ) and the straight dashed-line bond ( 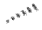 ) are used to represent the relative configuration of a stereogenic center. The wavy line ( 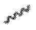 ) is used to represent a wedge-shaped solid-line bond ( 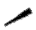 ) or a wedge-shaped dashed-line bond ( 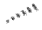 ), or the wavy line ( 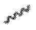 ) is used to represent a straight solid-line bond ( 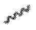 ) and a straight dashed-line bond ( 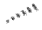 ).

The compounds of the present disclosure may have specific tautomeric forms. Unless otherwise specified, the term "tautomer" or "tautomeric form" refers to each of two or more isomers in which different isomeric forms of a functional group are in dynamic equilibrium and may readily convert to each other at room temperature. If tautomers possibly exist (for example, exist in a solution), a chemical equilibrium between the tautomers may be achieved. For example, proton tautomers (also referred to as prototropic tautomers) involve interconversions via proton migration, such as keto-enol isomerization and imine-enamine isomerization. Valence tautomers involve interconversions via recombination of some bonding electrons. Among them, a specific example of keto-enol tautomerization is the interconversion between the following two tautomers: pentane-2,4-dione and 4-hydroxypent-3-en-2-one.

Unless otherwise specified, when a compound of the present disclosure has isomers, there are cases where the compound is "enriched in one isomer", "isomerically enriched", "enriched in one enantiomer" or "enantiomerically enriched". The terms "enriched in one isomer", "isomerically enriched", "enriched in one enantiomer" or "enantiomerically enriched" mean that the content of one of the isomers or enantiomers is less than 100%, and the content of this isomer or enantiomer is greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise specified, when a compound of the present disclosure has isomers, there are cases of "isomeric excess" or "enantiomeric excess". The term "isomeric excess" or "enantiomeric excess" refers to the difference between the relative percentages of two isomers or two enantiomers. For example, if the content of one isomer or enantiomer is 90% and the content of the other isomer or enantiomer is 10%, then the isomeric or enantiomeric excess (ee value) is 80%.

The optically active (*R*)- and (*S*)-isomers and *D-* and *L-* isomers may be prepared by chiral synthesis, or with chiral reagents, or by other conventional techniques. If an enantiomer of a certain compound of the present disclosure is desired, it may be prepared by asymmetric synthesis or derivatization that uses a chiral auxiliary, in which the resulting mixture of diastereomers is separated and the auxiliary group is cleaved to provide the desired pure enantiomer. Alternatively, when the molecule contains a basic functional group (such as amino) or an acidic functional group (such as carboxyl), a salt of the diastereomer is formed by the molecule and an appropriate optically active acid or base, then diastereomeric resolution is performed by conventional methods well known in the art, and the pure enantiomer is obtained by recovery. In addition, the separation between enantiomer and diastereomer is usually accomplished by using chromatography, which adopts a chiral stationary phase and is optionally combined with a chemical derivatization method (for example, the formation of carbamate from amine). The compounds of the present disclosure may contain an atomic isotope in an unnatural proportion at one or more atoms constituting such compounds. For example, the compounds may be labeled with a radioisotope, such as tritium (³H), iodine-125 (¹²⁵I) or C-14 (¹⁴C). For another example, hydrogen may be substituted with heavy hydrogen to form a deuterated drug. The bond formed by deuterium and carbon is stronger than the bond formed by ordinary hydrogen and carbon. Compared with an undeuterated drug, a deuterated drug has advantages such as reduced toxicity and side effects, increased drug stability, strengthened efficacy, and prolonged biological half-life of drugs. All variations of the isotopic composition of the compounds of the present disclosure are included within the scope of the present disclosure regardless of the radioactivity.

The term "pharmaceutical composition" refers to a mixture consisting of one or more of the active ingredients or therapeutic combinations thereof of the present disclosure and a pharmaceutically acceptable excipient. The pharmaceutical composition is intended to facilitate the administration of the compound or the therapeutic combination thereof to a subject.

The term "pharmaceutically acceptable carrier" refers to any preparation or carrier medium capable of delivering an effective amount of the active substance of the present disclosure without interfering with the biological activity of the active substance, and exerts no toxic or side effects on the host or patient. Representative carriers include water, oils, vegetables and minerals, cream bases, lotion bases, ointment bases, etc. Such bases include suspending agents, tackifiers, penetration enhancers, and the like. Their preparations are well known to those skilled in the cosmetics field or the field of drugs for topical administration.

The term "excipient" generally refers to a carrier, a diluent and/or a medium required for the formulation of an effective pharmaceutical composition.

The wording "comprise" and the variants thereof, such as "comprises" or "comprising", should be understood as having an open and non-exclusive meaning, namely, "including but not limited to".

The term "treating" means administering a compound or a preparation described in the present application to ameliorate or eliminate a disease or one or more symptoms associated with the disease, and includes:
(i) inhibiting a disease or a disease state, i.e., restraining its development; and
(ii) alleviating a disease or a disease state, i.e., causing the regression of the disease or the disease state.

As for a drug or a pharmacologically active agent, the term "effective amount" or "therapeutically effective amount" refers to a sufficient amount of the drug or medicament that is not toxic but yet capable of achieving the intended effect. For the oral dosage form in the present disclosure, an "effective amount" of one active substance in a composition is the amount required to achieve the intended effect when used in combination with another active substance in the composition. The determination of an effective amount varies from person to person, depending on the age and the general condition of the subject as well as the specific active substance. An appropriate effective amount in a case may be determined by a person skilled in the art based on routine tests.

The terms "administer", "administration" and "administering" refer to physically introducing the composition comprising a therapeutic agent to an entity using any of a variety of methods and delivery systems known to those skilled in the art. Routes of administration of immune checkpoint inhibitors (e.g., an anti-PD-1 antibody or an anti-PD-L1 antibody) include intravenous, intramuscular, subcutaneous, intraperitoneal, spinal, or other parenteral routes of administration, for example, by injection or infusion. The phrase "parenteral administration" used herein refers to modes of administration apart from enteral and local administration, typically by injection, including but not limited to, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion and *in vivo* electroporation. In some embodiments, the immune checkpoint inhibitor (e.g., an anti-PD-1 antibody or an anti-PD-L1 antibody) is administered by a non-parenteral route, and in some embodiments, by oral administration. Other non-parenteral routes include local, epidermal or mucosal routes of administration, for example, intranasal, vaginal, rectal, sublingual or local administration. Administration may also be performed, e.g., once, multiple times, and/or over one or more extended periods of time.

The term "subject" refers to a mammal. In some embodiments, the subject is a mouse. In some embodiments, the subject is a human.

As used herein, "combined use" or "use in combination" means that two or more active substances may be administered to a subject simultaneously or sequentially in any order as a single formulation.

The term "active ingredient", "therapeutic agent", "active substance" or "active agent" refers to a chemical entity that may be effective in treating a target disorder, disease or condition.

"Optional" or "optionally" means that the event or situation described later may occur, but not necessarily, and such description includes a case where the event or situation occurs and a case where the event or situation does not occur.

The term "substituted" means that any one or more of the hydrogen atom(s) on a specific atom are replaced with a substituent, which may include heavy hydrogen or variants of hydrogen, as long as the valence state of the specific atom is normal and the substituted compound is stable. When the substituent is oxygen (i.e., =O), it means that two hydrogen atoms are substituted. Oxo does not occur on an aromatic group. The term "optionally substituted" means that it may or may not be substituted. Unless otherwise specified, the type and number of substituents may be arbitrary as long as the substitution is chemically feasible.

When any variable (such as R and 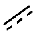 ) appears more than once in the composition or the structure of a compound, its definition in each case is independent. Thus, for example, if a group is substituted with 0 to 2 R, the group may optionally be substituted with up to two R, and there are independent options for R in each case. In addition, any combination of variables and/or variants thereof is allowed only in a case where such combination results in a stable compound.

When the number of a linking group is zero, such as -(CRR)₀-, it means that the linking group is a single bond.

When one of the variables is selected from a single bond, it means that the two groups linked by this variable are directly linked to each other. For example, when L in A-L-Z represents a single bond, the structure is actually A-Z.

When a substituent is absent, it means that the substituent does not exist. For example, when X in A-X is absent, it means that the structure is actually A. When a substituent may be linked to one or more atoms of a ring, such substituent may be bonded to any atom of the ring. For example, the structural unit 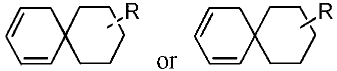 means a structural unit in which any position of cyclohexyl or cyclohexadiene may be substituted with the substituent R. When it is not specified that via which atom a listed substituent is linked to a substituted group, such substituent may be bonded via any atoms thereof. For example, pyridyl as a substituent may be linked to the substituted group via any carbon atom of the pyridine ring. When the linking direction of a listed linking group is not specified, the linking direction thereof is arbitrary. For example, when the linking group L in 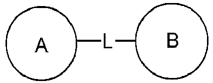 is -M-W-, the ring A and the ring B may be linked by -M-W- to either form 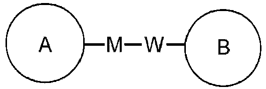 in the direction same as the left-to-right reading order, or form 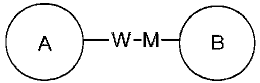 in the direction opposite to the left-to-right reading order. Combinations of the linking groups, substituents, and/or variants thereof are allowed only in a case where such combinations result in stable compounds. Unless otherwise specified, the term "hetero" means a heteroatom or a heteroatom radical (i.e., a heteroatom-containing radical), including atoms other than carbon (C) and hydrogen (H), and radicals containing these heteroatoms, such as oxygen (O), nitrogen (N), sulfur (S), silicon (Si), germanium (Ge), aluminum (Al), boron (B), -O-, -S-, =O, =S, -C(=O)O-, -C(=O)-, -C(=S)-, -S(=O), -S(=O)₂-, and optionally substituted -C(=O)N(H)-, -N(H)-, -C(=NH)-, -S(=O)₂N(H)or -S(=O)N(H)-.

The term "heteroalkyl" *per se* or in combination with another term refers to a stable linear or branched alkyl radical consisting of a certain number of carbon atoms and at least one heteroatom, or a combination thereof. In a typical example, the heteroatom is selected from B, O, N and S, wherein the nitrogen atom and the sulfur atom are optionally oxidized, and the nitrogen heteroatom is optionally quaternized. The heteroatom or heteroatom radical may be located at any internal position of the heterohydrocarbyl, including the position where the hydrocarbyl is linked to the rest moiety of a molecule. However, the terms "alkoxy", "alkylamino" and "alkylthio" (or thioalkoxy) are used by convention and refer to those alkyl groups that are linked to the rest moiety of a molecule via an oxygen atom, an amino group, or a sulfur atom, respectively. Examples include, but are not limited to -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -CH₂-CH=N-OCH₃ and -CH=CH-N(CH₃)-CH₃. At most two heteroatoms may appear consecutively, for example, -CH₂-NH-OCH₃.

Unless otherwise specified, the term "heterocycloalkyl" *per se* or in combination with other terms refers to a cyclized "heteroalkyl". In addition, in the case of "heterocycloalkyl", a heteroatom may occupy the position where the heterocycloalkyl is linked to the rest moiety of a molecule. In some embodiments, the heterocycloalkyl is a 4- to 6-membered heterocycloalkyl; in some other embodiments, the heterocycloalkyl is a 5- to 6-membered heterocycloalkyl. Examples of heterocycloalkyl include, but are not limited to, azetidinyl, oxetanyl, thietanyl, pyrrolidinyl, pyrazolidinyl, imidazolidinyl, tetrahydrothienyl, tetrahydrofuranyl, tetrahydropyranyl, piperidinyl, piperazinyl, morpholinyl, dioxanyl, dithianyl, isoxazolidinyl, isothiazolidinyl, 1,2-oxazinyl, 1,2-thiazinyl, hexahydropyridazinyl, homopiperazinyl, homopiperidinyl, oxepanyl,

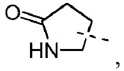

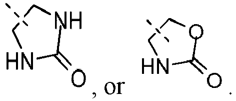

Unless otherwise specified, the term "alkyl" is used to represent a linear or branched saturated hydrocarbyl, which may be mono-substituted (such as -CH₂F) or poly-substituted (such as -CF₃), and may be monovalent (such as methyl), divalent (such as methylene) or polyvalent (such as methine). Examples of alkyl include methyl (Me), ethyl (Et), propyl (e.g., *n*-propyl and isopropyl), butyl (e.g., *n*-butyl, isobutyl, s-butyl and *t*-butyl), pentyl (e.g., *n*-pentyl, isopentyl and neopentyl), etc.

Unless otherwise specified, the term "halo" or "halogen" *per se* or as a part of another substituent means a fluorine atom, a chlorine atom, a bromine atom, or an iodine atom. In addition, the term "haloalkyl" is intended to include a monohaloalkyl and a polyhaloalkyl. For example, the term "halo(C₁-C₄) alkyl" is intended to include, but is not limited to, trifluoromethyl, 2,2,2-trifluoroethyl, 4-chlorobutyl, 3-bromopropyl, and the like. Unless otherwise specified, examples of haloalkyl include, but are not limited to: trifluoromethyl, trichloromethyl, pentafluoroethyl, and pentachloroethyl.

Unless otherwise specified, the term "heteroaryl" refers to an aryl (or an aromatic ring) containing one to four heteroatoms. In an exemplary example, the heteroatom is selected from B, N, O, and S, wherein the nitrogen atom and the sulfur atom are optionally oxidized, and the nitrogen atom is optionally quaternized. A heteroaryl may be attached to the rest moiety of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furanyl, thienyl, pyridyl, pyrimidinyl, benzothiazolyl, purinyl, benzoimidazolyl, indolyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furanyl, 3-furanyl, 2-thienyl, 3-thienyl, 2-pyridyl, 3-pyridyl, 4-pyridyl, 2-pyrimidinyl, 4-pyrimidinyl, 5-benzothiazolyl, purinyl, 2-benzoimidazolyl, 5-indolyl, 1-isoquinolyl, 5-isoquinolyl, 2-quinoxalinyl, 5-quinoxalinyl, 3-quinolyl, and 6-quinolyl.

Unless otherwise specified, C_{n - n+m} or Cₙ -Cₙ₊ₘ includes any specific case in which the number of the carbon atom is from n to n+m (for example, C₁₋₁₂ includes C₁, C₂, C₃, C₄, C₅, C₆, C₇, C₈, C₉, C₁₀, C₁₁, and C₁₂), and also includes any range between n and n+m (for example, C₁₋₁₂ includes C₁₋₃, C₁₋₆, C₁₋₉, C₃₋₆, C₃₋₉, C₃₋₁₂, C₆₋₉, C₆₋₁₂, C₉₋₁₂, etc.). Similarly, n-membered to n + m-membered means that the number of the atoms in a ring is from n to n+m (for example, a 3-to 12-membered ring includes a 3-membered ring, a 4-membered ring, a 5-membered ring, a 6-membered ring, a 7-membered ring, a 8-membered ring, a 9-membered ring, a 10-membered ring, a 11-membered ring, and a 12-membered ring), and also includes any range between n and n + m (for example, a 3- to 12-membered ring includes a 3- to 6-membered ring, a 3- to 9-membered ring, a 5- to 6-membered ring, a 5- to 7-membered ring, a 6- to 7-membered ring, a 6- to 8-membered ring, a 6- to 10-membered rings, *etc.).*

The term "antibody" refers to a binding protein having at least one antigen binding domain. The antibody and the fragment thereof of the present disclosure may be an intact antibody or any fragment thereof. Thus, the antibody and the fragment thereof of the present disclosure include a monoclonal antibody or a fragment thereof and an antibody variant or a fragment thereof, as well as an immunoconjugate. Examples of the antibody fragment include a Fab fragment, a Fab' fragment, an F(ab)' fragment, a Fv fragment, an isolated CDR region, a single chain Fv molecule (scFv), and other antibody fragments known in the art. The antibody and the fragment thereof may also include a recombinant polypeptide, a fusion protein, and a bispecific antibody. The anti-PD-Ll antibody and the fragment thereof disclosed herein may be of IgG1, IgG2, IgG3, or IgG4 isotype. The term "isotype" refers to the class of antibodies encoded by the heavy chain constant region gene. In one embodiment, the anti-PD-Ll antibody and the fragment thereof disclosed herein are of IgG1 or IgG4 isotype. The anti-PD-Ll antibody and the fragment thereof of the present disclosure can be derived from any species, including but not limited to, mouse, rat, rabbit, primate, llama, and human. The anti-PD-Ll antibody and the fragment thereof may be a murine antibody, a chimeric antibody, a humanized antibody or an intact human antibody. In one embodiment, the anti-PD-Ll antibody is an antibody produced by a hybridoma cell line derived from a mouse. Thus, in one embodiment, the anti-PD-Ll antibody is a murine antibody. In another embodiment, the anti-PD-Ll antibody is a chimeric antibody. In another embodiment, the chimeric antibody is a mouse-human chimeric antibody. In another embodiment, the antibody is a humanized antibody. In another embodiment, the antibody is derived from a murine antibody and is humanized.

The compounds of the present disclosure may be prepared by a variety of synthetic methods well known to a person skilled in the art, including the specific embodiments listed below, embodiments formed by combining the specific embodiments with other chemical synthesis methods, and equivalent alternatives well known to those skilled in the art. Preferred embodiments include, but are not limited to, the examples of the present disclosure.

The solvents used in the present disclosure are commercially available.

The following abbreviations are used in the present disclosure: DMF stands for *N*,*N*-dimethylformamide; DMA stands for *N*,*N*-dimethylacetamide; TEA stands for triethylamine; DIPEA stands for *N*,*N*-diisopropylethylamine; Pd(dppf)Cl₂ stands for [1,1'-bis(diphenylphosphino) ferrocene]palladium dichloride; Pd₂(dba)₃ stands for tris(dibenzylideneacetone)dipalladium; DPPF stands for 1,1'-bisdiphenylphosphinoferrocene; NBS stands for *N*-bromosuccinimide; POCl₃ stands for phosphorus oxychloride; HOBt stands for 1-hydroxybenzotriazole; HATU stands for 2-(7-oxybenzotriazol)-*N*,*N*,*N'*,*N'*-tetramethyluronium hexafluorophosphate; EDCI stands for 1-(3-dimethylaminopropyl)-3-ethylcarbodiimide hydrochloride; DIAD stands for diisopropyl azodicarboxylate; Boc₂O stands for di-*tert*-butyl dicarbonate; and ODPH stands for *O*-diphenylphosphinylhydroxylamine.

### DETAILED DESCRIPTION

The present disclosure is described in detail below by Examples, but it does not imply any disadvantageous limitation on the present disclosure. The present disclosure has been described in detail herein, and its specific embodiments are also disclosed therein. Various changes and improvements made to the specific embodiments of the present disclosure without departing from the spirit and scope of the present disclosure will be apparent to a person skilled in the art. 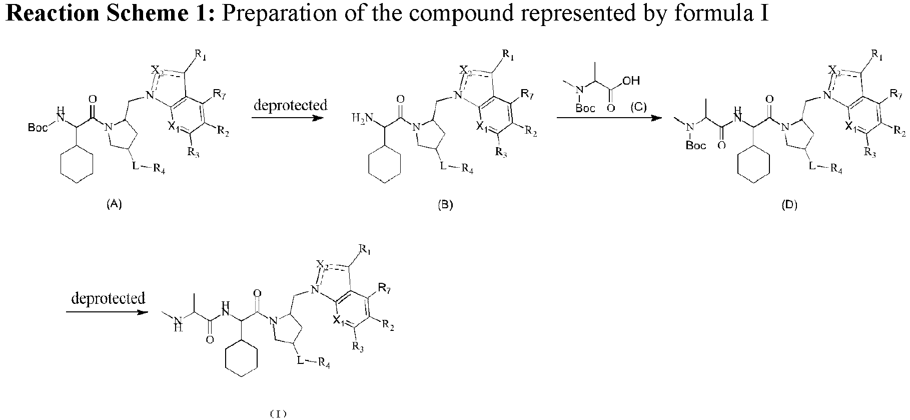 wherein 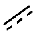 , X₁, X₂, L, R₁, R₂, R₃, R₄ and R₇ are as defined herein.

In the reactions shown in Reaction Scheme 1, Compound (A) is deprotected under an acidic condition (such as a hydrogen chloride/ethyl acetate solution) to obtain Compound (B); Compound (B) and Compound (C) undergo an acid-amine condensation reaction to obtain Compound (D), and this reaction requires a suitable condensing agent (such as HOBt), a suitable dehydrating agent (such as EDCI) and a suitable base (such as DIPEA) according to Reaction Scheme 1; and Compound (D) is then deprotected under an acidic condition (such as a hydrogen chloride/ethyl acetate solution) to obtain the compound represented by formula (I). 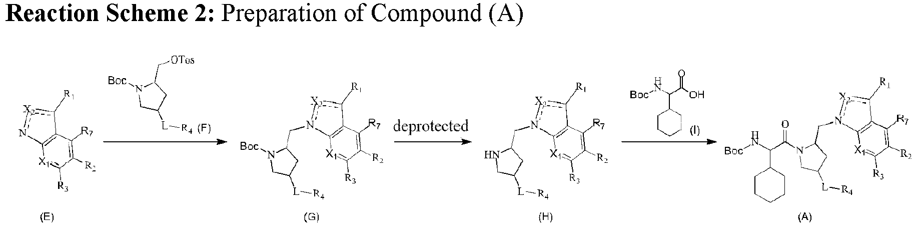 wherein 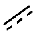 , X₁, X₂, L, R₁, R₂, R₃, R₄ and R₇ are as defined herein, and X₂ is not CCl.

In the reactions shown in Reaction Scheme 2, Compound (G) may be prepared via a substitution reaction between Compound (E) and Compound (F), this reaction requires a suitable base (such as potassium carbonate) and is preferably carried out at a high temperature according to Reaction Scheme 2; Compound (G) is deprotected under an acidic condition (such as a hydrogen chloride/ethyl acetate solution) to obtain Compound (H); and Compound (A) may be prepared via an acid-amine condensation reaction between Compound (H) and Compound (I), and this reaction requires a suitable condensing agent (such as HOBt), a suitable dehydrating agent (such as EDCI) and a suitable base (such as DIPEA). 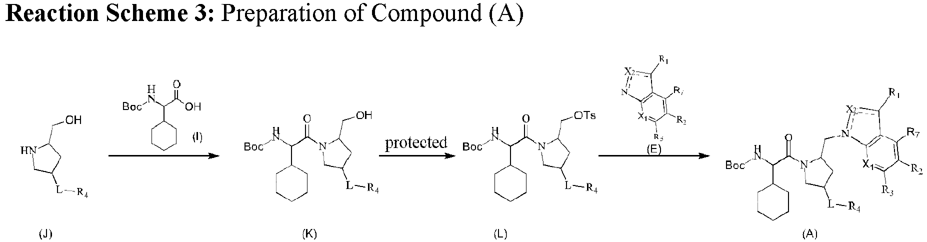 wherein X₁ is N, X₂ is CCl; and 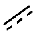 , L, R₁, R₂, R₃, R₄ and R₇ are as defined herein.

In the reactions shown in Reaction Scheme 3, Compound (K) may be prepared via an acid-amine condensation reaction between Compound (J) and Compound (I), and this reaction requires a suitable condensing agent (such as HATU) and a suitable base (such as DIPEA); Compound (K) is reacted with p-toluenesulfonyl chloride under a basic condition (such as TEA) to obtain Compound (L); and Compound (A) may be prepared via a substitution reaction between Compound (L) and Compound (E) under a basic condition (such as potassium carbonate), and according to Reaction Scheme 2, this reaction is preferably carried out at a high temperature. 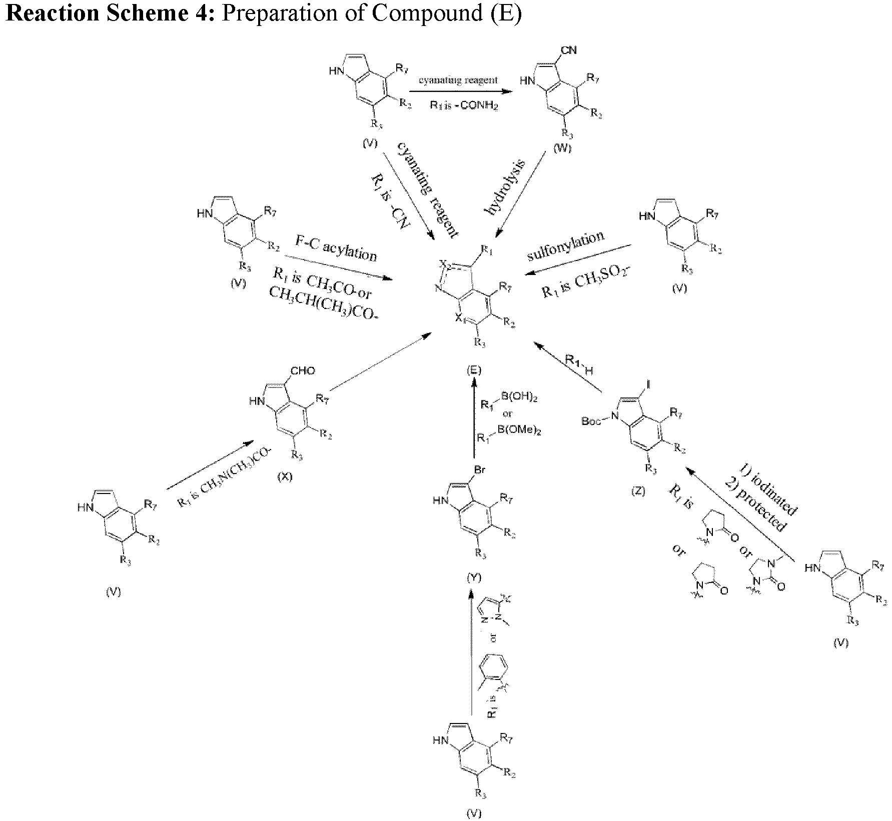 wherein X₁ is CH, X₂ is CH; and 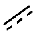 , L, R₂, R₃, R₄ and R₇ are as defined herein.

In the reactions shown in Reaction Scheme 4, when R₁ is CH₃CO- or CH₃CH(CH₃)CO-, Compound (E) may be prepared via an F-C acylation reaction between Compound (V) and the corresponding acyl halide or anhydride, and this reaction requires a suitable catalyst (such as aluminum trichloride).

When R₁ is -CN, Compound (E) may be prepared by reacting Compound (V) with a cyanating reagent, this reaction requires a suitable cyanating reagent (such as chlorosulfonyl isocyanate), and according to Reaction Scheme 4, this reaction is preferably carried out at a low temperature.

When R₁ is -CONH₂, Compound (V) is reacted with a cyanating reagent to give Compound (W), this reaction requires a suitable cyanating reagent (such as chlorosulfonyl isocyanate), and according to Reaction Scheme 4, this reaction is preferably carried out at a low temperature; Compound (E) may be prepared via a hydrolysis reaction of Compound (W) under a basic condition, and this reaction requires a suitable base (such as potassium carbonate) and a suitable solvent (such as an ethanol/hydrogen peroxide mixed solvent).

When R₁ is CH₃SO₂-, Compound (E) may be prepared via a sulfonylation reaction between Compound (V) and methanesulfonyl chloride under a basic condition, this reaction requires a suitable base (such as potassium *tert*-butoxide) and a suitable catalyst (such as a solution of triethylborane in tetrahydrofuran), and according to Reaction Scheme 4, this reaction is preferably carried out at a low temperature.

When R₁ is CH₃N(CH₃)CO-, a Vilsmeier-Haack reaction of Compound (V) with POCl₃ and DMF gives Compound (X). Compound (E) may be prepared by reacting Compound (X) with dimethylamine, and this reaction requires a suitable catalyst (such as sodium cyanide) and a suitable oxidant (such as manganese dioxide).

When R₁ is 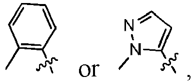 a halogenation reaction between Compound (V) and a brominating reagent gives Compound (Y), and this reaction requires a suitable brominating reagent (such as NBS). Compound (E) may be prepared via a Suzuki coupling reaction of Compound (Y) with the corresponding boric acid or boric acid ester, this reaction requires a suitable catalyst (such as Pd(dppf)Cl₂) and a suitable base (such as potassium phosphate), and according to Reaction Scheme 4, this reaction is preferably carried out at a high temperature.

When R₁ is 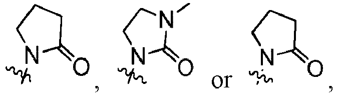 a halogenation reaction between Compound (V) and an iodinating reagent gives an intermediate compound and this reaction requires a suitable iodinating reagent (such as elemental iodine), and then the intermediate compound is reacted with Boc₂O to obtain Compound (Z) and this reaction requires a suitable catalyst (such as DMAP) and a suitable base (such as TEA). Compound (E) may be prepared via a Ullmann coupling reaction between Compound (Z) and the corresponding compound with a saturated five-membered aza-ring, this reaction requires a suitable catalyst (such as cuprous iodide), a suitable ligand (such as *N*,*N*-dimethylethylenediamine) and a suitable base (such as cesium carbonate), and according to Reaction Scheme 4, this reaction is preferably carried out at a high temperature.

### Example 1

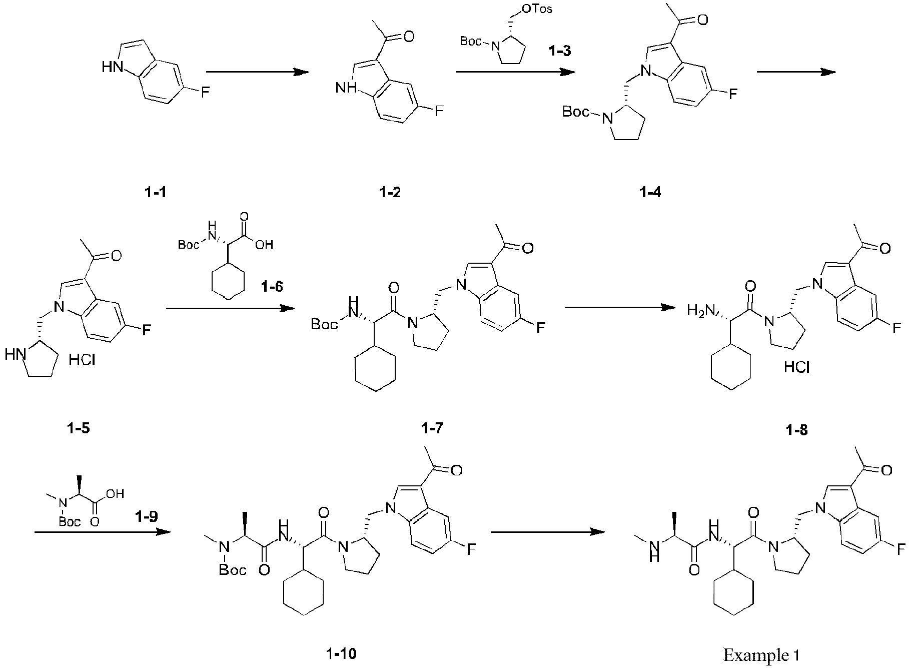

### Step 1:

Acetic anhydride (7.55 g, 74 mmol, 6.93 mL, 2.0 equiv.) was added dropwise into a suspension of ammonium chloride (7.92 g, 148 mmol, 5.17 mL, 4.0 equiv.) in 1,2-dichloroethane (100 mL) at 15 °C. The mixture was stirred at 15 °C for 30 minutes, and a solution of Compound 1-1 (5.0 g, 37 mmol, 1.0 equiv.) in 1,2-dichloroethane (50 mL) was added into the mixture. The resulting mixture was stirred at 15 °C for 2 hours. Aluminum trichloride (9.87 g, 74 mmol, 2.0 equiv.) was added into the reaction solution, and the reaction solution was changed from heterogeneous to homogeneous. Acetic anhydride (3.78 g, 37 mmol, 3.47 mL, 1.0 equiv.) was further added into the reaction solution, and the reaction solution was stirred at 15 °C for 30 minutes. LCMS showed that the starting materials were reacted completely. The reaction solution was slowly poured into ice water (200 mL), and the resulting mixture was extracted with ethyl acetate (100 mL × 2). The combined organic phases were washed with saline (100 mL), then the resulting mixture was subjected to liquid-liquid separation, and the resultant was concentrated under reduced pressure. The resulting residue was purified by silica gel column chromatography (petroleum ether : ethyl acetate = 10 : 1 to 1 : 1) to obtain Compound 1-2. LCMS (ESI) m/z: 178.1 (M+1).

### Step 2:

Compound 1-3 (7.11 g, 20.01 mmol, 3.0 equiv.) and potassium carbonate (4.61 g, 33.35 mmol, 5.0 equiv.) were added into a solution of Compound 1-2 (1.36 g, 6.67 mmol, 1.0 equiv.) in DMF (20 mL). Under the protection of nitrogen, the resulting mixture was heated to 100 °C and reacted for 15 hours. LCMS showed that the starting materials were not reacted completely. The reaction solution was heated to 120 °C and reacted for 2 hours. LCMS showed that the reaction was complete. Water (30 mL) was added into the reaction solution, and the resulting mixture was extracted with ethyl acetate (30 mL × 3). The combined organic phases were washed with saline (30 mL) and then concentrated, and the resulting residue was purified by silica gel column chromatography (petroleum ether : ethyl acetate = 10 : 1 to 3 : 1) to obtain Compound 1-4. LCMS (ESI) m/z: 361.1 (M+1).

### Step 3:

A hydrogen chloride/ethyl acetate solution (4.0 mol/L, 20 mL, 21.57 equiv.) was added into a solution of Compound 1-4 (2.0 g, 3.71 mmol, 1.0 equiv.) in ethyl acetate (20 mL) at 0 °C. After the resulting reaction solution was stirred at 15 °C for 1 hour, a large amount of solid precipitated, and LCMS showed that the reaction was complete. The reaction solution was filtered, and the filter cake was washed with ethyl acetate (10 mL) and then dried to obtain Compound 1-5. The crude product was directly used in the next step. ¹H NMR (400MHz, DMSO-*d*₆) δ 8.75 (s, 1H), 7.90-7.79 (m, 2H), 7.17 (dt, *J* = 9.2, 2.6 Hz, 1H), 4.84-4.58 (m, 2H), 3.36-3.20 (m, 1H), 3.19-3.04 (m, 1H), 2.44 (d, *J* = 2.8 Hz, 4H), 2.18-2.06 (m, 1H), 2.02 (br dd, *J=* 7.9, 5.5 Hz, 1H), 1.88 (td, *J=* 12.6, 7.9 Hz, 1H), 1.80-1.67 (m, 1H); LCMS (ESI) m/z: 261.1 (M+1).

### Step 4:

Compound 1-6 (1.30 g, 5.05 mmol, 1.5 equiv.), HOBt (500.85 mg, 3.71 mmol, 1.1 equiv.), EDCI (710.56 mg, 3.71 mmol, 1.1 equiv.) and DIPEA (1.31 g, 10.11 mmol, 1.76 mL, 3 equiv.) were added into a solution of Compound 1-5 (1.0 g, 3.37 mmol, 1.0 equiv.) in dichloromethane (30 mL), and the resulting mixture was reacted at 15 °C for 16 hours. LCMS showed that the reaction was complete. The reaction solution was poured into water (50 mL), and the resulting mixture was extracted with dichloromethane (50 mL × 3). The combined organic phases were concentrated, and the resulting residue was purified by silica gel column chromatography (petroleum ether : ethyl acetate = 10 : 1 to 1 : 1) to obtain Compound 1-7. LCMS (ESI) m/z: 500.2 (M+1).

### Step 5:

Hydrogen chloride/ethyl acetate (4.0 mol/L, 20 mL, 23.61 equiv.) was added into a solution of Compound 1-7 (1.75 g, 3.39 mmol, 1.0 equiv.) in ethyl acetate (20 mL) at 0 °C. The mixture was reacted at 15 °C for 1 hour, and LCMS showed that the reaction was complete. The reaction solution was concentrated to obtain Compound 1-8, and the crude product was directly used in the next step. LCMS (ESI) m/z: 400.1 (M+1).

### Step 6:

Compound 1-9 (1.05 g, 5.16 mmol, 1.5 equiv.), HOBt (511.41 mg, 3.78 mmol, 1.1 equiv.), EDCI (725.54 mg, 3.78 mmol, 1.1 equiv.) and DIPEA (1.33 g, 10.32 mmol, 1.8 mL, 3 equiv.) were added into a solution of Compound 1-8 (1.5 g, 3.44 mmol, 1.0 equiv.) in dichloromethane (30 mL), and the resulting reaction solution was reacted at 15 °C for 14 hours. LCMS showed that the reaction was complete. The reaction solution was poured into water (50 mL), the resulting mixture was extracted with dichloromethane (50 mL × 3), and the combined organic phases were concentrated. The resulting residue was purified by silica gel column chromatography (petroleum ether : ethyl acetate = 2 : 1 to 1 : 4) to obtain Compound 1-10. LCMS (ESI) m/z: 585.3 (M+1).

### Step 7:

Hydrogen chloride/ethyl acetate (4.0 mol/L, 18.57 mL, 33.41 equiv.) was added into a solution of Compound 1-10 (1.30 g, 2.22 mmol, 1.0 equiv.) in ethyl acetate (20 mL) at 0 °C. The resulting mixture was reacted at 15 °C for 1 hour. LCMS showed that the reaction was complete. The reaction solution was concentrated, and the resulting residue was purified by preparative HPLC (hydrochloric acid system, mobile phase: water (0.05% hydrochloric acid)acetonitrile, gradient: acetonitrile: 15% to 25%) to obtain the hydrochloride of Example 1. ¹H NMR (400MHz, CD₃OD) δ 8.28 (s, 1H), 7.87 (dd, *J=* 9.7, 2.4 Hz, 1H), 7.78 (dd, *J* = 8.9, 4.2 Hz, 1H), 7.04 (dt, *J* = 9.0, 2.4 Hz, 1H), 4.56-4.42 (m, 3H), 4.14-4.02 (m, 1H), 3.95 (q, *J* = 6.8 Hz, 1H), 3.83 (q, *J* = 8.4 Hz, 1H), 3.77-3.66 (m, 1H), 2.67 (s, 3H), 2.50 (s, 3H), 2.25-2.09 (m, 1H), 2.03-1.93 (m, 1H), 1.85-1.64 (m, 9H), 1.51 (d, *J* = 7.0 Hz, 3H), 1.33-1.01 (m, 6H); LCMS (ESI) m/z: 485.2 (M+1).

### Example 2

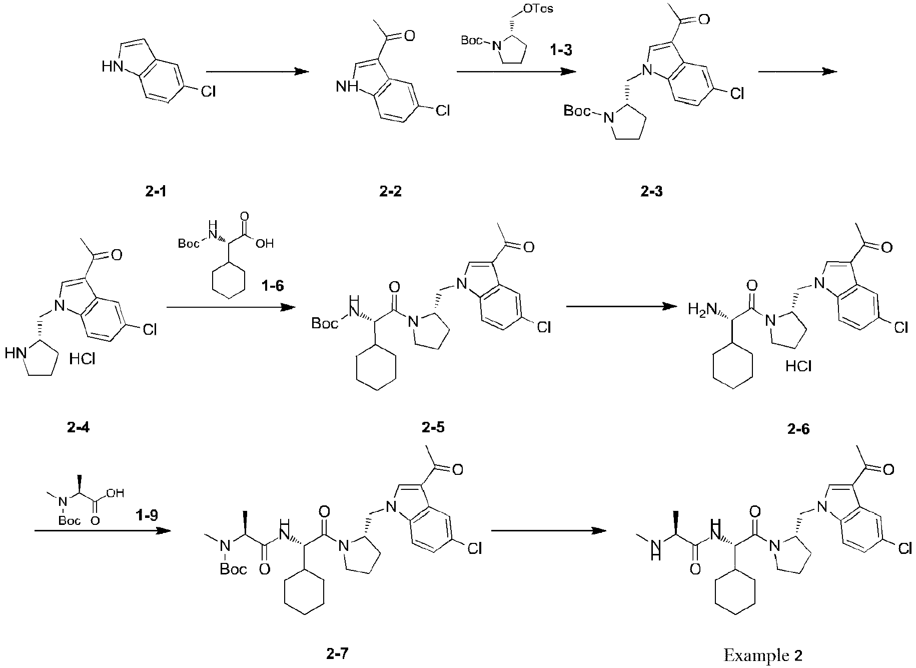

### Step 1:

Acetic anhydride (6.73 g, 65.97 mmol, 6.18 mL, 2.0 equiv.) was added into a suspension of ammonium chloride (3.53 g, 65.97 mmol, 2.31 mL, 2.0 equiv.) in 1,2-dichloroethane (10 mL) at 15 °C, and the mixture was stirred at 15 °C for 15 minutes. Compound 2-1 (5.0 g, 32.98 mmol, 1.0 equiv.) was added into the mixture, and the resulting mixture was stirred at 15 °C for 2 hours. Aluminum trichloride (8.80 g, 65.97 mmol, 2.0 equiv.) was added into the reaction solution, and the reaction solution was stirred at 15 °C for 30 minutes. Acetic anhydride (3.37 g, 32.98 mmol, 3.09 mL, 1.0 equiv.) was further added into the reaction solution, and the reaction solution was stirred at 15 °C for 15 minutes. LCMS showed that the starting materials were reacted completely. The reaction solution was slowly poured into ice water, and the resulting mixture was extracted with ethyl acetate (100 mL × 3). The extract liquor was dried over Na₂SO₄ and concentrated under reduced pressure. The resulting residue was purified by silica gel column chromatography (petroleum ether : ethyl acetate = 1 : 1) to obtain Compound 2-2. LCMS (ESI) m/z: 194.1 (M+1).

### Step 2:

Compound 2-2 (2.00 g, 10.33 mmol, 1.0 equiv.) and potassium carbonate (7.14 g, 51.65 mmol, 5.0 equiv.) were added into a solution of Compound 1-3 (7.54 g, 20.66 mmol, 2.0 equiv.) in DMF (70 mL), and the mixture was heated and stirred for 12 hours at 100 °C. Water (300 mL) and ethyl acetate (300 mL) were added into the reaction solution, and the organic phase was washed with saline (100 mL), dried over Na₂SO₄, filtered, and concentrated under reduced pressure to obtain Compound 2-3. LCMS (ESI) m/z: 377.0 (M+1).

### Step 3:

A hydrogen chloride/dioxane solution (4.0 mol/L, 20 mL, 26.04 equiv.) was added into a solution of Compound 2-3 (2.4 g, 3.07 mmol, 1.0 equiv.) in dioxane (20 mL), and the resulting reaction solution was stirred at 15 °C for 10 hours. LCMS showed that the reaction was complete. The reaction solution was filtered, and the filter cake was washed with ethyl acetate (10 mL × 3) and then dried to obtain Compound 2-4. The crude product was directly used in the next step. LCMS (ESI) m/z: 277.1 (M+1).

### Step 4:

DIPEA (857.20 mg, 6.63 mmol, 1.16 mL, 3 equiv.) and HATU (1.01 g, 2.65 mmol, 1.2 equiv.) were added into a solution of Compound 1-6 (625.81 mg, 2.43 mmol, 1.1 equiv.) in DMF (5 mL), and the mixture was stirred at 15 °C for 30 minutes. Compound 2-4 (700 mg, 2.21 mmol, 1.0 equiv., hydrochloride) was added into the reaction solution, and the reaction mixture was stirred at 15 °C for 1.5 hours. Water (30 mL) and ethyl acetate (40 mL) were added into the reaction solution. The organic phase was washed with citric acid (20 mL, 10% aqueous solution) and saline (20 mL), dried over Na₂SO₄, filtered, and concentrated under reduced pressure to obtain Compound 2-5. LCMS (ESI) m/z: 516.2 (M+1).

### Step 5:

Hydrogen chloride/dioxane (4.0 mol/L, 18.33 mL, 34.40 equiv.) was added into a solution of Compound 2-5 (1.10 g, 2.13 mmol, 1.0 equiv.) in dioxane (10 mL), and the mixture was reacted at 15 °C for 1.5 hours. The reaction solution was filtered, and the filter cake was washed with ethyl acetate (20 mL) and then dried to obtain Compound 2-6. LCMS (ESI) m/z: 416.2 (M+1).

### Step 6:

DIPEA (326.91 mg, 2.53 mmol, 440.58 µL, 3 equiv.), HATU (384.71 mg, 1.01 mmol, 1.2 equiv.) and Compound 2-6 (500 mg, 843.16 µmol, 1.0 equiv., hydrochloride) were added into a solution of Compound 1-9 (188.50 mg, 927.48 µmol, 1.1 equiv.) in DMF (5 mL), and the reaction mixture was stirred at 15 °C for 1 hour. Water (30 mL) and ethyl acetate (20 mL) were added into the reaction solution. The organic phase was washed with citric acid (20 mL, 10% aqueous solution) and saline (20 mL), dried over Na₂SO₄, filtered, and concentrated under reduced pressure to obtain Compound 2-7. The crude product was directly used in the next step. LCMS (ESI) m/z: 601.1 (M+1).

### Step 7:

Trifluoroacetic acid (3 mL) was added into a solution of Compound 2-7 (500 mg, 787.28 µmol, 1.0 equiv.) in dichloromethane (10 mL) at 0 °C, and the resulting mixture was reacted at 0 °C for 1 hour. LCMS showed that the reaction was complete. The reaction solution was concentrated, and the resulting residue was purified by preparative HPLC (hydrochloric acid) to obtain the hydrochloride of Example 2. LCMS (ESI) m/z: 501.4 (M+1). ¹H NMR (400MHz, DMSO-*d₆*) δ 9.50 (br s, 1H), 8.88 (br d, *J* = 5.3 Hz, 1H), 8.78 (d, *J* = 8.2 Hz, 1H), 8.47 (s, 1H), 8.15 (d, *J* = 2.1 Hz, 1H), 7.89 (d, *J* = 8.8 Hz, 1H), 7.32 (dd, *J* = 2.1, 8.7 Hz, 1H), 4.48-4.33 (m, 3H), 4.10 (br dd, *J* = 14.8, 9.9 Hz, 1H), 3.90-3.90 (m, 1H), 3.73-3.54 (m, 2H), 2.46-2.45 (m, 1H), 2.44 (s, 3H), 2.16-2.01 (m, 1H), 1.97-1.81 (m, 1H), 1.79-1.52 (m, 9H), 1.34 (d, *J=* 6.8 Hz, 3H), 1.27-0.87 (m, 6H).

### Example 3

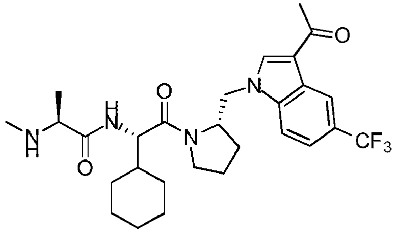

Please refer to Example 1 for the preparation method of Example 3. ¹H NMR (400MHz, CD₃OD) δ 8.57 (s, 1H), 8.41 (s, 1H), 7.97 (d, *J* = 8.7 Hz, 1H), 7.55 (dd, *J* = 8.7, 1.6 Hz, 1H), 4.64-4.54 (m, 2H), 4.48 (d, *J* = 7.7 Hz, 1H), 4.25-4.12 (m, 1H), 3.95-3.81 (m, 2H), 3.79-3.69 (m, 1H), 2.67 (s, 3H), 2.55 (s, 3H), 2.26-2.12 (m, 1H), 2.09-1.97 (m, 1H), 1.91-1.59 (m, 9H), 1.50 (d, *J* = 7.0 Hz, 3H), 1.36-0.97 (m, 6H); LCMS (ESI) m/z : 535.2 (M+1).

### Example 4

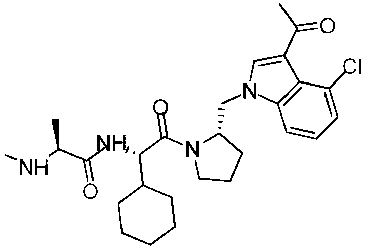

Please refer to Example 1 for the preparation method of Example 4. ¹H NMR (400MHz, DMSO-*d₆*) δ 9.32 (br s, 1H), 8.85 (br d, *J* = 5.9 Hz, 1H), 8.78 (br d, *J* = 8.2 Hz, 1H), 8.45 (s, 1H), 7.88 (d, *J* = 7.1 Hz, 1H), 7.31-7.20 (m, 2H), 4.48-4.36 (m, 3H), 4.14-4.04 (m, 1H), 3.92-3.81 (m, 1H), 3.73-3.64 (m, 1H), 3.63-3.56 (m, 1H), 2.49-2.45 (m, 4H), 2.17-2.02 (m, 1H), 1.96-1.84 (m, 1H), 1.81-1.56 (m, 9H), 1.34 (d, *J* = 6.8 Hz, 3H), 1.27-0.95 (m, 6H); LCMS (ESI) m/z : 501.4 (M+1).

### Example 5

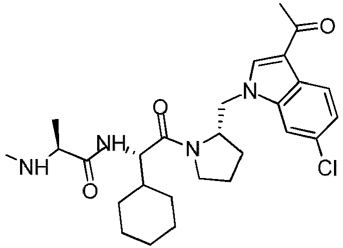

Please refer to Example 1 for the preparation method of Example 5. ¹H NMR (400MHz, DMSO-*d₆*) δ 9.50 (br s, 1H), 8.96-8.85 (m, 1H), 8.82 (d, *J* = 8.3 Hz, 1H), 8.45 (s, 1H), 8.15 (d, *J =* 8.5 Hz, 1H), 8.00 *(d, J* = 1.6 Hz, 1H), 7.24 (dd, *J* = 8.5, 1.8 Hz, 1H), 4.47-4.35 (m, 3H), 4.08 (dd, *J* = 14.5, 9.9 Hz, 1H), 3.89-3.80 (m, 1H), 3.74-3.66 (m, 1H), 3.64-3.56 (m, 1H), 2.46 (br s, 1H), 2.44 (s, 3H), 2.18-2.05 (m, 1H), 1.96-1.84 (m, 1H), 1.79-1.57 (m, 9H), 1.35 (d, *J* = 6.8 Hz, 3H), 1.27-0.95 (m, 6H); LCMS (ESI) m/z: 501.4 (M+1).

### Example 6

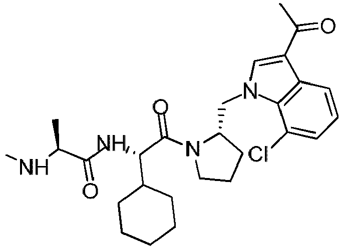

Please refer to Example 1 for the preparation method of Example 6. ¹H NMR (400MHz, DMSO-*d₆*) δ 9.30 (br s, 1H), 8.80 (br s, 1H), 8.59 (br d, *J* = 7.8 Hz, 1H), 8.39 (br s, 1H), 8.19 (br d, *J* = 7.8 Hz, 1H), 7.37-7.07 (m, 2H), 4.87-4.59 (m, 2H), 4.57-4.29 (m, 2H), 3.79 (br s, 1H), 3.65 (br s, 2H), 2.43 (br s, 7H), 2.16-1.23 (m, 13H), 1.21-0.73 (m, 5H); LCMS (ESI) m/z: 501.3 (M+1).

### Example 7

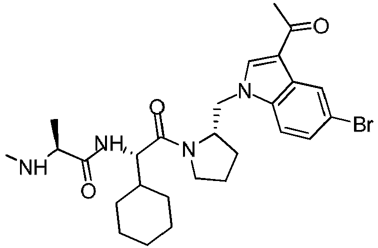

Please refer to Example 1 for the preparation method of Example 7. ¹H NMR (400MHz, DMSO-*d₆*) δ 9.60-9.44 (m, 1H), 8.97-8.84 (m, 1H), 8.79 (br d, *J* = 8.2 Hz, 1H), 8.46 (s, 1H), 8.31 (d, *J* = 1.8 Hz, 1H), 7.84 (d, *J* = 8.8 Hz, 1H), 7.43 (dd, *J* = 8.8, 2.0 Hz, 1H), 4.47-4.35 (m, 3H), 4.18-3.98 (m, 1H), 3.85 (br d, *J =* 4.8 Hz, 1H), 3.73-3.64 (m, 1H), 2.47-2.42 (m, 6H), 2.17-2.02 (m, 1H), 1.98-1.85 (m, 1H), 1.81-1.52 (m, 9H), 1.34 (d, *J* = 6.8 Hz, 3H), 1.21-0.98 (m, 5H); LCMS (ESI) m/z: 547.2 (M+1).

### Example 8

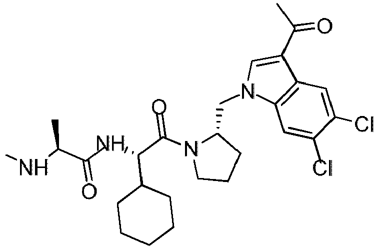

Please refer to Example 1 for the preparation method of Example 8. ¹H NMR (400MHz, DMSO-*d*₆) δ 9.60-9.48 (m, 1H), 8.96-8.83 (m, 1H), 8.79 (d, *J* = 8.3 Hz, 1H), 8.53 (s, 1H), 8.28 (s, 1H), 8.20 (s, 1H), 4.48-4.32 (m, 3H), 4.11 (br dd, *J=* 13.3, 8.2 Hz, 1H), 3.83-3.77 (m, 1H), 3.73-3.65 (m, 1H), 3.64-3.52 (m, 1H), 2.47-2.43 (m, 6H), 2.21-2.03 (m, 1H), 1.96-1.84 (m, 1H), 1.77-1.72 (m, 2H), 1.67-1.55 (m, 5H), 1.34 (d, *J=* 6.8 Hz, 3H), 1.26-0.86 (m, 6H); LCMS (ESI) m/z: 535.3 (M+1).

### Example 9

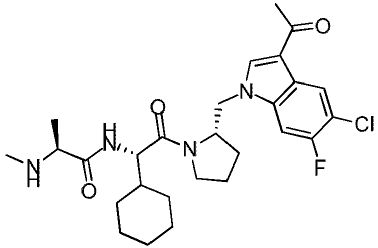

Please refer to Example 1 for the preparation method of Example 9. ¹H NMR (400 MHz, DMSO-*d*₆) δ 0.89-1.27 (m, 6 H) 1.34 (d, *J* = 6.85 Hz, 3H), 1.58-1.70 (m, 5H), 1.73-1.80 (m, 2H), 1.86-1.97 (m, 1 H), 2.05-2.19 (m, 1H), 2.42-2.47 (m, 6H), 3.50-3.74 (m, 2H), 3.79-3.91 (m, 1H), 4.11 (dd, *J* = 13.75, 8.50 Hz, 1H), 4.26-4.46 (m, 3H), 7.98 (d, *J* = 10.15 Hz, 1H), 8.19-8.24 (m, 1H), 8.49 (s, 1H), 8.75 (d, *J* = 8.19 Hz, 1H), 8.86 (br d, *J* = 5.62 Hz, 1H), 9.43 (br s, 1H); LCMS (ESI) m/z: 519.3 (M+1).

### Example 10

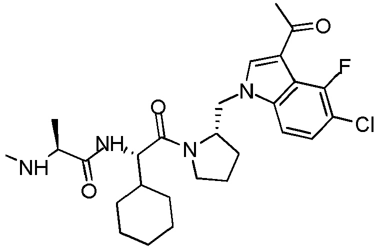

Please refer to Example 1 for the preparation method of Example 10. ¹H NMR (400MHz, DMSO-*d*₆) δ 9.63-9.35 (m, 1H), 8.88 (br d, *J =* 5.3 Hz, 1H), 8.76 (br d, *J* = 8.1 Hz, 1H), 8.55-8.43 (m, 1H), 7.73 (d, *J* = 8.8 Hz, 1H), 7.41 (dd, *J* = 8.4, 6.7 Hz, 1H), 4.47-4.29 (m, 3H), 4.19-4.05 (m, 1H), 3.95-3.78 (m, 1H), 3.16 (s, 3H), 2.47-2.43 (m, 5H), 2.21-2.02 (m, 1H), 1.90 (br d, *J* = 3.3 Hz, 1H), 1.84-1.46 (m, 9H), 1.33 (br d, *J* = 6.7 Hz, 3H), 1.22-0.91 (m, 5H); LCMS (ESI) m/z: 519.3 (M+1).

### Example 11

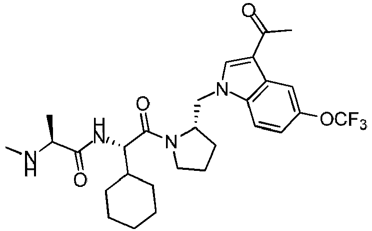

Please refer to Example 1 for the preparation method of Example 11. ¹H NMR (400 MHz, DMSO-*d*₆) δ 0.96-1.19 (m, 5H), 1.30-1.42 (m, 3H), 1.53-1.80 (m, 1H), 1.53-1.82 (m, 7H), 1.90 (br s, 1H), 2.02-2.17 (m, 1H), 2.41-2.48 (m, 6H), 3.57-3.63 (m, 1H), 3.83-3.91 (m, 1 H), 4.07-4.19 (m, 1H), 4.37-4.46 (m, 2H), 7.28 (br d, *J* = 8.19 Hz, 1H), 7.97 (d, *J=* 8.93 Hz, 1H), 8.03-8.13 (m, 1H), 8.55 (s, 1H), 8.79 (br d, *J* = 7.95 Hz, 1H), 8.90 (br s, 1H), 9.60 (br d, *J* = 5.01 Hz, 1H); LCMS (ESI) m/z: 551.3 (M+1).

### Example 12

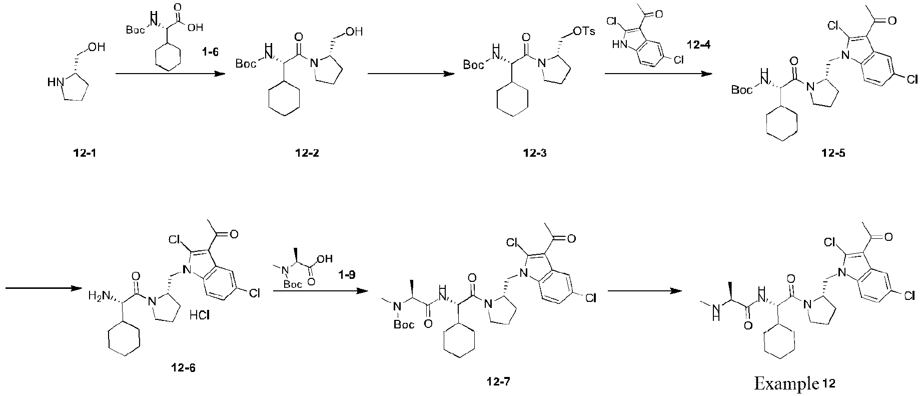

### Step 1:

Compound 1-6 (2.54 g, 9.89 mmol, 1.0 equiv.), HATU (3.76 g, 9.89 mmol, 1.0 equiv.) and DIPEA (3.83 g, 29.66 mmol, 5.17 mL, 3.0 equiv.) were added into a solution of Compound 12-1 (1.0 g, 9.89 mmol, 0.96 mL, 1.0 equiv.) in dichloromethane (20 mL). The mixture was reacted at 30 °C for 2.0 hours. TLC (petroleum ether : ethyl acetate = 1 : 1) detection showed that the starting materials were reacted completely. The reaction solution was poured into water (100 mL), the mixture was extracted with ethyl acetate (50 mL × 2), and the combined organic phases were concentrated. The resulting residue was purified by silica gel column chromatography (petroleum ether : ethyl acetate = 5 : 1 to 1 : 1) to obtain Compound 12-2. ¹H NMR (400MHz, CDCl₃) δ 5.21 (br d, *J* = 9.0 Hz, 1H), 4.75 (br d, *J* = 6.4 Hz, 1H), 4.31-4.18 (m, 2H), 3.95-3.78 (m, 1H), 3.69-3.61 (m, 1H), 3.60-3.52 (m, 1H), 3.47 (td, *J* = 10.2, 7.3 Hz, 1H), 2.12-2.03 (m, 2H), 1.96-1.80 (m, 3H), 1.69-1.51 (m, 5H), 1.41 (s, 9H), 1.22-0.97 (m, 6H); LCMS (ESI) m/z: 341.2 (M+1).

### Step 2:

TEA (1.60 g, 15.86 mmol, 2.21 mL, 3.0 equiv.) and *p*-toluenesulfonyl chloride (1.21 g, 6.34 mmol, 1.20 equiv.) were added into a solution of Compound 12-2 (1.80 g, 5.29 mmol, 1.0 equiv.) in dichloromethane (40 mL). The resulting mixture was reacted at 30 °C for 2.0 hours. LCMS detection showed that the starting materials were not reacted completely. Water (100 mL) was added to the reaction solution, and the resulting mixture was subjected to liquid-liquid separation. The obtained organic phase was washed with saturated saline (100 mL) and concentrated to obtain Compound 12-3. The crude product was used directly in the next step. LCMS (ESI) m/z: 495.3 (M+1).

### Step 3:

Please refer to the preparation method of Compound 1-4 for the preparation method of Compound 12-5. LCMS (ESI) m/z: 550.2 (M+1).

### Step 4:

Please refer to the preparation method of Compound 1-8 for the preparation method of Compound 12-6. LCMS (ESI) m/z: 450.2 (M+1).

### Step 5:

Please refer to the preparation method of Compound 1-10 for the preparation method of Compound 12-7. LCMS (ESI) m/z: 635.3 (M+1).

### Step 6:

Please refer to Example 1 for the preparation method of the hydrochloride of Example 12. ¹H NMR (400MHz, DMSO-*d*₆) δ 9.31 (br s, 1H), 8.87 (br d, *J* = 8.2 Hz, 2H), 8.21 (d, *J* = 1.6 Hz, 1H), 7.93 (d, *J=* 8.8 Hz, 1H), 7.40 (dd, *J=* 8.8, 1.7 Hz, 1H), 4.57-4.29 (m, 3H), 4.28-4.11 (m, 1H), 3.82 (br s, 2H), 2.60 (s, 3H), 2.11-1.93 (m, 2H), 1.81-1.51 (m, 8H), 1.44-0.90 (m, 12H); LCMS (ESI) m/z: 535.2 (M+1).

### Example 13

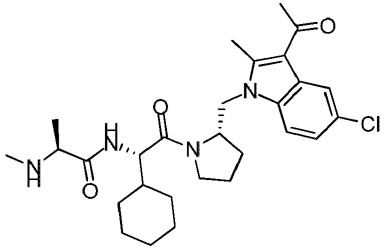

Please refer to Example 1 for the preparation method of the hydrochloride of Example 13. ¹H NMR (400 MHz, DMSO-*d*₆) δ 0.92-1.16 (m, 5H), 1.33 (br d, *J* = 6.72 Hz, 3H), 1.47-1.68 (m, 8H), 1.91 (br d, *J* = 6.48 Hz, 1H), 2.16-2.32 (m, 1H), 2.44 (br t, *J*=5.01 Hz, 3H), 2.53 (s, 3H), 2.80 (s, 3H), 3.59-3.63 (m, 1H), 3.65-3.72 (m, 1H), 3.85 (br dd, *J* = 11.55, 6.79 Hz, 1H), 4.17-4.45 (m, 4H), 7.23 (dd, J= 8.68, 1.59 Hz, 1H), 7.80 (d, *J=* 8.80 Hz, 1H), 8.02 (d, *J=* 1.71 Hz, 1H), 8.73 (br d, *J* = 8.19 Hz, 1H), 8.86 (br s, 1H), 9.45 (br s, 1H); LCMS (ESI) m/z : 515.2 (M+1).

### Example 14

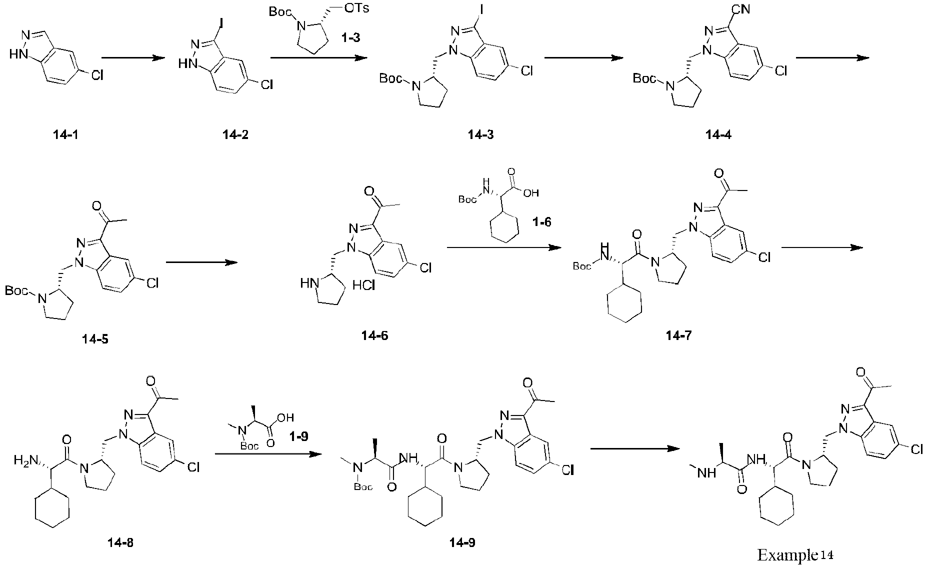

### Step 1:

Iodine (13.31 g, 52.43 mmol, 10.56 mL, 2.0 equiv.) and potassium hydroxide (5.88 g, 104.86 mmol, 4 equiv.) were added into a solution of Compound 14-1 (4 g, 26.22 mmol, 1.0 equiv.) in DMA (100 mL) at 0 °C. The resulting mixture was reacted at 20 °C for 12 hours. LCMS showed that the reaction was complete. A saturated aqueous sodium sulfite solution (200 mL) was added into the reaction solution, and the resulting mixture was extracted with ethyl acetate (200 mL × 2). The combined organic phases were washed with saturated saline (200 mL) and then concentrated. The resulting residue was slurried with petroleum ether (5 mL) to obtain Compound 14-2. ¹H NMR (400MHz, DMSO-*d*₆) δ 13.71 (br s, 1H), 7.60 (d, *J* = 8.8 Hz, 1H), 7.47-7.38 (m, 2H).

### Step 2:

Please refer to the preparation method of Compound 1-4 for the preparation method of Compound 14-3. ¹H NMR (400MHz, DMSO-*d*₆) δ 7.66 (d, *J=* 8.9 Hz, 1H), 7.54-7.41 (m, 2H), 4.56-4.41 (m, 2H), 4.23-4.05 (m, 1H), 3.29-3.01 (m, 2H), 1.93-1.57 (m, 4H), 1.37 (br s, 5H), 1.11 (br s, 4H); LCMS (ESI) m/z: 484.1 (M+23).

### Step 3:

Under the protection of nitrogen, zinc cyanide (0.72 g, 6.12 mmol, 0.388 mL, 0.6 equiv.), Pd₂(dba)₃ (0.93 g, 1.02 mmol, 0.1 equiv.), zinc powder (1.33 g, 20.42 mmol, 2.0 equiv.) and DPPF (1.13 g, 2.04 mmol, 0.2 equiv.) were added into a solution of Compound 14-3 (4.80 g, 10.21 mmol, 1.0 equiv.) in DMF (100 mL). The resulting mixture was heated to 100 °C and reacted for 2 hours. LCMS showed that the reaction was complete. The reaction solution was cooled and then filtered. The filter cake was washed with ethyl acetate (50 mL) and the filtrate was concentrated. The resulting residue was purified by silica gel column chromatography (petroleum ether : ethyl acetate = 10 : 1 to 3 : 1) to obtain Compound 14-4. LCMS (ESI) m/z: 383.2 (M+23).

### Step 4:

Methylmagnesium bromide (3 mol/L, 1.48 mL, 2 equiv.) was added into a solution of Compound 14-4 (0.8 g, 2.22 mmol, 1 equiv.) in tetrahydrofuran (20 mL) at 0 °C, and the resulting mixture was reacted at 20 °C for 2 hours. LCMS showed that the reaction was complete. The reaction solution was slowly poured into water (100 mL), and the mixture was extracted with ethyl acetate (50 mL × 2). The combined organic phases were washed with saturated saline (100 mL), then the resulting mixture was subjected to liquid-liquid separation, and the organic phase was concentrated to obtain Compound 14-5. The crude product was used directly in the next step. LCMS (ESI) m/z: 378.1 (M+1).

### Step 5:

Please refer to the preparation method of Compound 1-5 for the preparation method of Compound 14-6. LCMS (ESI) m/z: 278.1 (M+1).

### Step 6:

Please refer to the preparation method of Compound 1-7 for the preparation method of Compound 14-7. LCMS (ESI) m/z: 539.4 (M+23).

### Step 7:

Please refer to the preparation method of Compound 1-8 for the preparation method of Compound 14-8. LCMS (ESI) m/z: 417.1 (M+1).

### Step 8:

Please refer to the preparation method of Compound 1-10 for the preparation method of Compound 14-9. LCMS (ESI) m/z: 624.3 (M+23).

### Step 9:

Please refer to Example 1 for the preparation method of Example 14. ¹H NMR (400MHz, DMSO-*d₆*) δ 9.06-8.79 (m, 2H), 8.70 (br d, *J* = 8.2 Hz, 1H), 8.17-8.10 (m, 1H), 7.97 (br d, *J* = 8.9 Hz, 1H), 7.56 (br d, *J* = 8.7 Hz, 1H), 4.71 (br dd, *J* = 13.2, 3.8 Hz, 1H), 4.58-4.44 (m, 2H), 4.38 (br t, *J* = 7.6 Hz, 1H), 3.85 (br d, *J* = 5.1 Hz, 2H), 2.62 (s, 3H), 1.92-1.74 (m, 5H), 1.72-1.49 (m, 8H), 1.31 (br d, *J* = 6.7 Hz, 3H), 1.13 (br d, *J* = 13.7 Hz, 4H), 1.04-0.91 (m, 2H); LCMS (ESI) m/z: 502.1 (M+1).

### Example 15

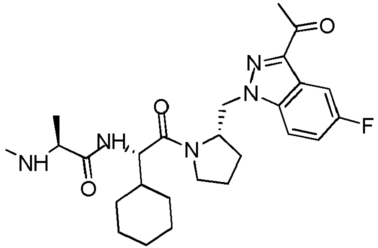

Please refer to Example 14 for the preparation method of Example 15. ¹H NMR (400MHz, DMSO-*d₆*) δ 9.56 (br s, 1H), 8.87 (br s, 1H), 8.80-8.60 (m, 1H), 8.14-7.94 (m, 1H), 7.85-7.68 (m, 1H), 7.58-7.40 (m, 1H), 4.78-4.30 (m, 4H), 3.96-3.74 (m, 1H), 3.64-3.58 (m, 2H), 2.61 (s, 1H), 2.47-2.38 (m, 3H), 1.94-1.52 (m, 11H), 1.39-1.26 (m, 3H), 1.24-0.87 (m, 6H); LCMS (ESI) m/z: 486.3 (M+1).

### Example 16

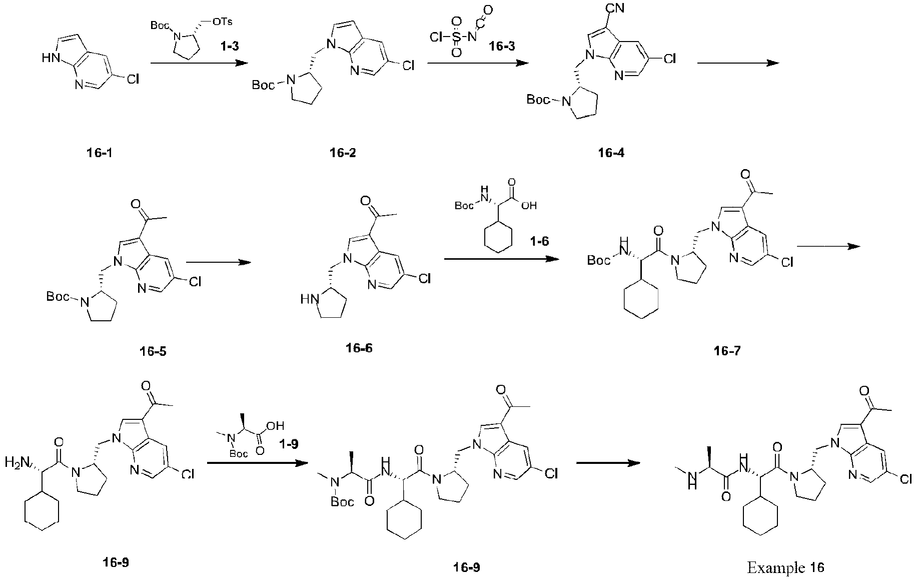

### Step 1:

Please refer to the preparation method of Compound 1-4 for the preparation method of Compound 16-2. LCMS (ESI) m/z: 336.2 (M+1).

### Step 2:

Compound 16-3 (997.86 mg, 7.05 mmol, 612.18 µL, 1.5 equiv.) was added into a solution of Compound 16-2 in DMF (20 mL) at -20 °C, and the resulting mixture was stirred at 0 °C for 2 hours. TLC showed that the starting materials were reacted completely. 1.5 mL of Compound 16-3 (2.44 g, 17.28 mmol, 1.5 mL, 3.68 equiv.) was additionally added into the above reaction solution at -20 °C, and the resulting mixture was stirred at 0 °C for 0.5 hour. LCMS showed that the starting materials were reacted completely. Water (50 mL) was added into the reaction mixture, and the resulting mixture was filtered. The filter cake was washed with water (20 mL × 2) and then dried in vacuum. The resulting residue was purified by silica gel column chromatography (petroleum ether : ethyl acetate = 50 : 1 to 10 : 1) to obtain Compound 16-3. ¹H NMR (400 MHz, DMSO-*d*₆) δ 0.98 (br s, 5H), 1.14-1.31 (m, 4H), 1.80 (br d, *J=* 17.85 Hz, 4H), 3.17-3.31 (m, 2H), 4.12-4.30 (m, 2H), 4.35-4.44 (m, 1H), 8.24-8.39 (m, 1H), 8.42-8.57 (m, 2H); LCMS (ESI) m/z: 361.1 (M+1).

### Step 3:

Please refer to the preparation method of Compound 14-5 for the preparation method of Compound 16-5. LCMS (ESI) m/z: 378.2 (M+1).

### Step 4:

Please refer to the preparation method of Compound 1-5 for the preparation method of Compound 16-6. LCMS (ESI) m/z: 278.0 (M+1).

### Step 5:

Please refer to the preparation method of Compound 1-7 for the preparation method of Compound 16-7. LCMS (ESI) m/z: 539.4 (M+23).

### Step 6:

Please refer to the preparation method of Compound 1-8 for the preparation method of Compound 16-8. LCMS (ESI) m/z: 417.3 (M+1).

### Step 7:

Please refer to the preparation method of Compound 1-10 for the preparation method of Compound 16-9. LCMS (ESI) m/z: 624.1 (M+23).

### Step 8:

Please refer to Example 1 for the preparation method of Example 16. ¹H NMR (400 MHz, DMSO-*d*₆) δ 1.02-1.14 (m, 6H), 1.35-1.49 (m, 2H), 1.58 (br s, 4 H), 1.66-1.80 (m, 4H), 1.87 (br d, *J* = 6.72 Hz, 2H), 2.09-2.20 (m, 3H), 2.46 (s, 3H), 2.93 (dt, *J* = 13.57, 6.79 Hz, 1H), 3.54 (br d, *J* = 9.90 Hz, 2H), 3.62-3.70 (m, 1H), 4.21-4.41 (m, 2H), 4.48 (br dd, *J* = 13.51, 7.03 Hz, 1H), 4.68 (br d, *J* = 3.18 Hz, 1H), 7.80 (br d, *J* = 8.80 Hz, 1H), 8.32-8.40 (m, 1H), 8.40-8.47 (m, 1H), 8.55-8.66 (m, 1H); LCMS (ESI) m/z: 502.1 (M+1).

### Example 17

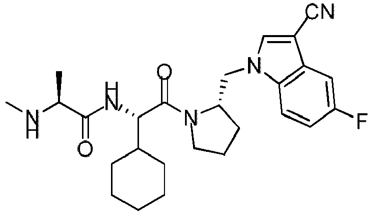

Please refer to Example 16 and Example 1 for the preparation method of Example 17. ¹H NMR (400MHz, CD₃OD) δ 8.53 (br d, *J* = 7.6 Hz, 1H), 8.06 (s, 1H), 7.88 (dd, *J* = 8.9, 4.2 Hz, 1H), 7.32 (dd, *J* = 8.8, 2.3 Hz, 1H), 7.14 (dt, *J* = 9.1, 2.3 Hz, 1H), 4.57-4.45 (m, 3H), 4.21-4.08 (m, 1H), 3.92 (q, *J* = 6.8 Hz, 1H), 3.86-3.78 (m, 1H), 3.76-3.65 (m, 1H), 2.67 (s, 3H), 2.22-2.07 (m, 1H), 2.04-1.96 (m, 1H), 1.83-1.63 (m, 8H), 1.50 (d, *J* = 6.8 Hz, 3H), 1.3-1.02 (m, 6H); LCMS (ESI) m/z: 468.2 (M+1).

### Example 18

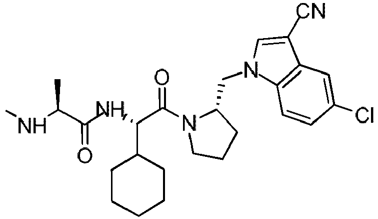

Please refer to Example 16 and Example 1 for the preparation method of Example 18. ¹H NMR (400MHz, DMSO-*d₆*) δ 9.60-9.41 (m, 1H), 8.86 (br d, *J* = 4.6 Hz, 1H), 8.69 (d, *J* = 8.3 Hz, 1H), 8.37 (s, 1H), 7.94 (d, *J* = 8.8 Hz, 1H), 7.66 (d, *J* = 1.8 Hz, 1H), 7.39 (dd, *J* = 8.8, 2.0 Hz, 1H), 4.41 (br d, *J* = 2.8 Hz, 1H), 4.38 (br d, *J* = 6.5 Hz, 1H), 4.23-4.12 (m, 1H), 3.88-3.79 (m, 1H), 3.70-3.53 (m, 2H), 2.44 (br t, *J* = 5.1 Hz, 3H), 2.15-2.01 (m, 1H), 1.90 (br d, *J* = 4.3 Hz, 1H), 1.75-1.52 (m, 8H), 1.32 (d, *J* = 6.8 Hz, 3H), 1.25-0.89 (m, 6H); LCMS (ESI) m/z: 484..4 (M+1).

### Example 19

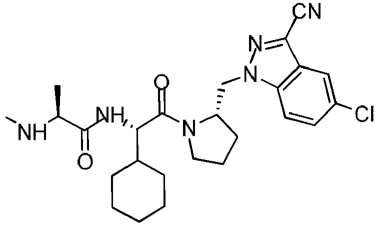

Please refer to Example 14 for the preparation method of Example 19. ¹H NMR (400MHz, CD₃OD) δ 7.93 (d, *J* = 9.0 Hz, 1H), 7.83 (d, *J* = 1.1 Hz, 1H), 7.54 (dd, *J* = 8.9, 1.5 Hz, 1H), 4.77-4.70 (m, 1H), 4.68-4.61 (m, 1H), 4.57 (br s, 1H), 4.48 (br d, *J* = 7.0 Hz, 1H), 3.88 (q, *J* = 7.0 Hz, 1H), 3.84-3.74 (m, 1H), 3.65-3.54 (m, 1H), 2.68-2.62 (m, 3H), 1.97-1.85 (m, 3H), 1.83-1.66 (m, 7H), 1.47 (d, *J* = 7.0 Hz, 3H), 1.37-1.00 (m, 5H); LCMS (ESI) m/z: 485.1 (M+1).

### Example 20

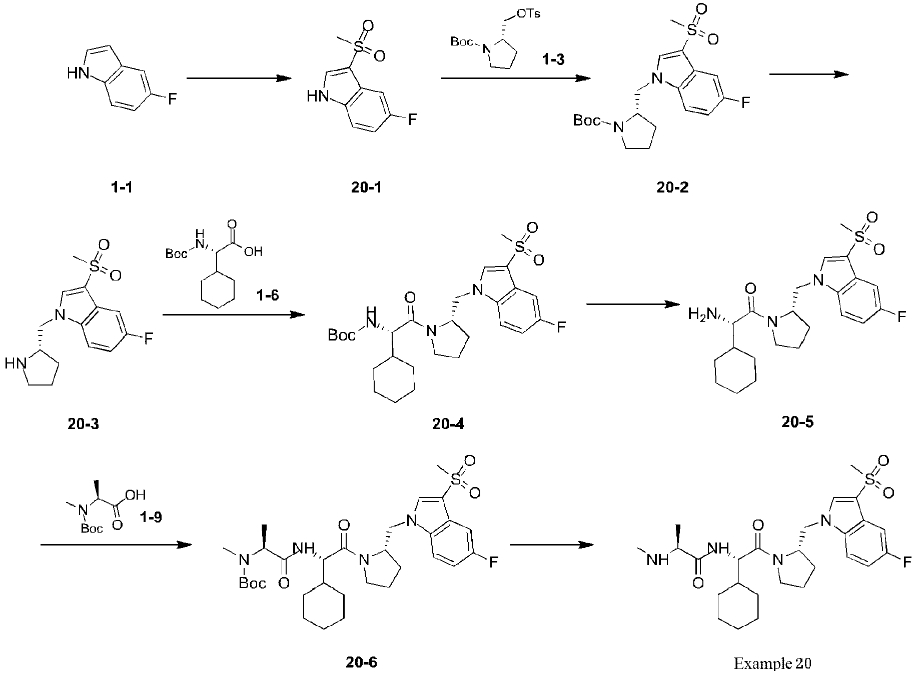

### Step 1:

Under the protection of nitrogen, potassium *tert*-butoxide (0.91 g, 8.14 mmol, 1.1 equiv.) was added into a solution of Compound 1-1 (1.0 g, 7.40 mmol, 1.0 equiv.) in tetrahydrofuran (20 mL). After the mixture was reacted at 15 °C for 0.5 hour, a solution of triethylborane in tetrahydrofuran (1 mol/L, 8.14 mL, 1.1 equiv.) was slowly added into the reaction solution. The reaction solution was further reacted at 15 °C for 0.5 hour, and methanesulfonyl chloride (0.93 g, 8.14 mmol, 0.63 mL, 1.1 equiv.) was then added into the reaction solution. The reaction solution was reacted at -15 °C for 10 hours. LCMS showed that the reaction was complete. A saturated aqueous ammonium chloride solution (100 mL) was added into the reaction system, the resulting mixture was extracted with ethyl acetate (100 mL × 2), and the combined organic phases were concentrated to obtain Compound 20-1. LCMS (ESI) m/z: 214.0 (M+1).

### Step 2:

Please refer to the preparation method of Compound 1-4 for the preparation method of Compound 20-2. LCMS (ESI) m/z: 397.1 (M+1).

### Step 3:

Please refer to the preparation method of Compound 1-5 for the preparation method of Compound 20-3. LCMS (ESI) m/z: 297.1 (M+1).

### Step 4:

Please refer to the preparation method of Compound 1-7 for the preparation method of Compound 20-4. LCMS (ESI) m/z: 558.1 (M+23).

### Step 5:

Please refer to the preparation method of Compound 1-8 for the preparation method of Compound 20-5.

### Step 6:

Please refer to the preparation method of Compound 1-10 for the preparation method of Compound 20-6. LCMS (ESI) m/z: 643.4 (M+23).

### Step 7:

Please refer to Example 1 for the preparation method of Example 20. ¹H NMR (400MHz, DMSO-*d₆*) δ 9.61-9.37 (m, 1H), 8.96-8.83 (m, 1H), 8.79 (d, *J=* 8.2 Hz, 1H), 8.23-8.19 (m, 1H), 7.98 (dd, *J* = 9.2, 4.4 Hz, 1H), 7.52 (dd, *J* = 9.4, 2.5 Hz, 1H), 7.26 (dt, *J* = 9.2, 2.5 Hz, 1H), 4.51-4.32 (m, 3H), 4.15 (dd, *J=* 13.2, 8.6 Hz, 1H), 3.98-3.79 (m, 1H), 3.20 (s, 3H), 2.46 (t, *J* = 5.3 Hz, 3H), 2.08 (td, *J* = 12.2, 8.7 Hz, 1H), 1.95-1.82 (m, 1H), 1.77-1.49 (m, 9H), 1.37-1.30 (m, 3H), 1.26-0.91 (m, 6H); LCMS (ESI) m/z: 521.3 (M+1).

### Example 21

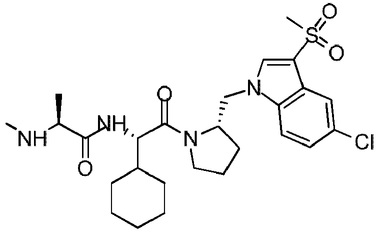

Please refer to Example 20 for the preparation method of Example 21. ¹H NMR (400MHz, CD₃OD) δ 8.03 (s, 1H), 7.91-7.80 (m, 2H), 7.32 (dd, *J* = 8.7, 1.9 Hz, 1H), 4.59-4.43 (m, 2H), 4.21-4.10 (m, 1H), 3.94 (q, *J* = 6.8 Hz, 1H), 3.82 (q, *J* = 8.5 Hz, 1H), 3.74-3.63 (m, 1H), 3.18 (s, 3H), 2.67 (s, 3H), 2.16-2.02 (m, 1H), 2.01-1.94 (m, 1H), 1.85-1.72 (m, 6H), 1.71-1.59 (m, 2H), 1.50 (d, *J=* 7.0 Hz, 3H), 1.34-1.01 (m, 6H); LCMS (ESI) m/z: 537.1 (M+1).

### Example 22

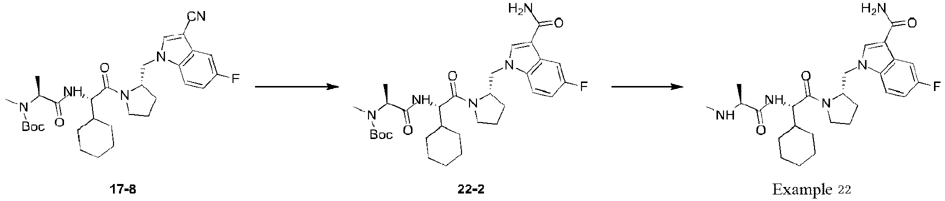

### Step 1:

Potassium carbonate (85.21 mg, 616.53 µmol, 2.0 equiv.) and hydrogen peroxide (9.44 g, 83.26 mmol, 8 mL, concentration: 30%, 270.09 equiv.) were added into a solution of Compound 17-8 (175 mg, 308.27 µmol, 1.0 equiv.) in ethanol (8 mL), and the resulting reaction solution was reacted at 50 °C for 1 hour. LCMS showed that the reaction was complete. Water (40 mL) was added into the reaction solution, and the resulting mixture was extracted with ethyl acetate (30 mL). The combined organic phases were washed with saturated saline (20 mL), dried over anhydrous sodium sulfate, and then filtered. The filtrate was concentrated under reduced pressure to obtain Compound 22-2. LCMS (ESI) m/z: 586.6 (M+1).

### Step 2:

Please refer to Example 1 for the preparation method of Example 22. ¹H NMR (400MHz, DMSO-*d₆*) δ 9.48 (br s, 1H), 8.88 (br s, 1H), 8.81 (br d, *J* = 7.9 Hz, 1H), 8.16 (s, 1H), 7.87-7.78 (m, 2H), 7.13-7.06 (m, 1H), 4.44-4.36 (m, 2H), 4.32 (br d, *J* = 4.1 Hz, 1H), 4.03 (br dd, *J* = 13.5, 9.6 Hz, 1H), 3.86 (br d, *J* = 4.9 Hz, 2H), 3.63-3.53 (m, 1H), 2.44 (br s, 1H), 2.15-2.02 (m, 1H), 1.91 (br s, 1H), 1.77-1.58 (m, 9H), 1.35 (br d, *J=* 6.8 Hz, 3H), 1.21-0.99 (m, 6H); LCMS (ESI) m/z: 486.5 (M+1).

### Example 23

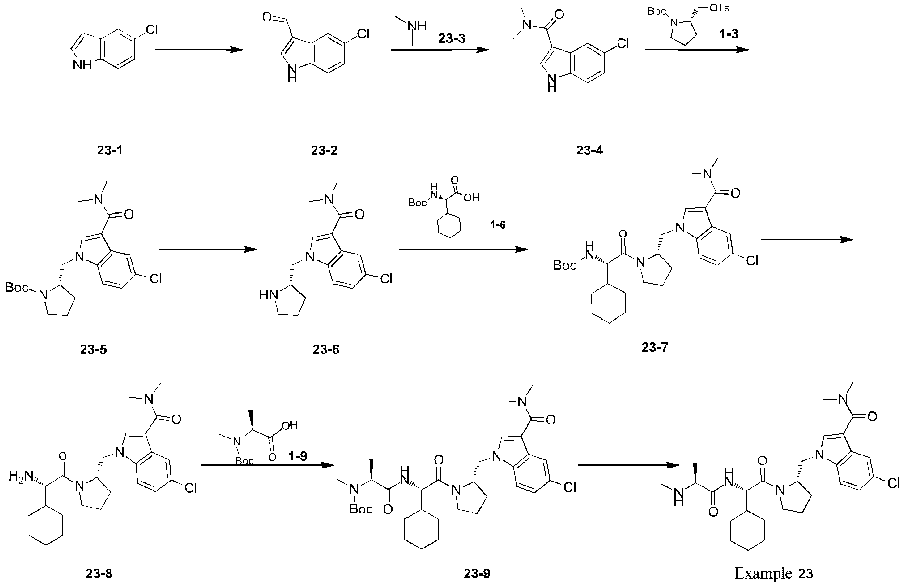

### Step 1:

POCl₃ (3.96 g, 25.83 mmol, 2.40 mL, 1.31 equiv.) was slowly added dropwise into a solution of DMF (8.55 g, 116.97 mmol, 9 mL, 5.91 equiv.) at 0 °C over 30 minutes. A solution of Compound 23-1 (3.0 g, 19.79 mmol, 1.0 equiv.) in DMF (3 mL) was added dropwise into the mixed solution, and the resulting mixture was stirred at 25 °C for 1 hour. TLC (petroleum ether: ethyl acetate = 3 : 1) and LCMS showed that the starting materials were reacted completely. The reaction solution was slowly poured into water (150 mL), the pH of the mixed solution was adjusted to 9 with a 10% NaOH solution, and then the resulting mixture was extracted with ethyl acetate (200 mL × 2). The combined organic phases were washed with saturated saline (100 mL × 2), dried over anhydrous sodium sulfate and then concentrated to obtain Compound 23-2. ¹H NMR (400 MHz, DMSO-*d*₆) δ 7.28 (dd, *J* = 8.66, 2.13 Hz, 1H), 7.54 (d, *J* = 8.66 Hz, 1H), 8.06 (d, *J* = 2.01 Hz, 1H), 8.36 (s, 1H), 9.93 (s, 1H), 12.29 (br s, 1H); LCMS (ESI) m/z: 180.1 (M+1).

### Step 2:

Compound 23-3 (2 mol/L, 33.41 mL, 4.0 equiv.) was added into a solution of Compound 23-2 (3 g, 16.70 mmol, 1.0 equiv.) and sodium cyanide (163.72 mg, 3.34 mmol, 0.2 equiv.) in DMF (30 mL), and the resulting mixture was stirred at 30 °C for 10 minutes. Manganese dioxide (36.30 g, 417.59 mmol, 25.0 equiv.) was added in portions into the mixture, and the resulting mixture was further stirred at 30 °C for 14 hours. LCMS showed that the starting materials were reacted completely. The reaction mixture was filtered, and the filter cake was washed with ethyl acetate (100 mL × 2). The combined organic phases were successively washed with a saturated ferrous sulfate solution (50 mL × 2) and saline (100 mL), dried over anhydrous sodium sulfate and then concentrated to obtain Compound 23-4. The crude product was used directly in the next step. LCMS (ESI) m/z: 223.2 (M+1).

### Step 3:

Please refer to the preparation method of Compound 1-4 for the preparation method of Compound 23-5. LCMS (ESI) m/z: 406.0 (M+1).

### Step 4:

Please refer to the preparation method of Compound 1-5 for the preparation method of Compound 23-6. ¹H NMR (400 MHz, DMSO-*d*₆) δ 1.62-1.77 (m, 1H), 1.80-1.92 (m, 1H), 1.94-2.04 (m, 1H), 2.06-2.17 (m, 1H), 3.04-3.15 (m, 6H) 3.20-3.31 (m, 1H), 3.86 (br s, 1H), 4.52-4.56 (m, 1H), 4.61 (br dd, *J=* 14.87, 5.08 Hz, 2H), 4.69-4.77 (m, 1H), 4.69-4.77 (m, 1H), 7.26 (dd, J= 8.72, 2.07 Hz, 1H), 7.79 (d, J= 8.78 Hz, 1H), 7.90 (d, *J=* 2.01 Hz, 1H), 8.26 (s, 1H), 9.35 (br s, 1H), 10.04 (br s, 1H); LCMS (ESI) m/z: 306.1(M+1).

### Step 5:

Please refer to the preparation method of Compound 1-7 for the preparation method of Compound 23-7. LCMS (ESI) m/z: 545.4 (M+1)

### Step 6:

Please refer to the preparation method of Compound 1-8 for the preparation method of Compound 23-8. LCMS (ESI) m/z: 445.0 (M+1).

### Step 7:

Please refer to the preparation method of Compound 1-10 for the preparation method of Compound 23-9. LCMS (ESI) m/z: 630.3 (M+1).

### Step 8:

Please refer to Example 1 for the preparation method of Example 23. ¹H NMR (400 MHz, CD₃OD) δ 1.00-1.30 (m, 5H), 1.47-1.54 (m, 3H), 1.61 (br d, *J* = 12.23 Hz, 1H), 1.65-1.87 (m, 7H), 1.99 (br s, 1H), 2.06-2.23 (m, 1H), 2.64-2.71 (m, 3H), 3.1-3.30 (m, 6H), 3.72 (br d, *J* = 6.48 Hz, 1H), 3.83 (q, *J* = 8.48 Hz, 1H), 3.90-4.02 (m, 1H), 4.09-4.20 (m, 1H), 4.46 (br d, *J* = 7.46 Hz, 1H), 4.51-4.62 (m, 2H), 7.24-7.30 (m, 1H), 7.74 (s, 1H), 7.80 (d, *J* = 9.05 Hz, 1H), 7.92 (br s, 1H); LCMS (ESI) m/z: 530.3(M+1).

### Example 24

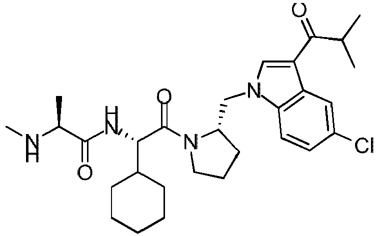

Please refer to Example 1 for the preparation method of Example 24. ¹H NMR (400MHz, CD₃OD) δ 8.36-8.23 (m, 2H), 7.79 (d, *J* = 8.8 Hz, 1H), 7.27 (dd, *J* = 8.8, 2.0 Hz, 1H), 4.60-4.44 (m, 3H), 4.21-4.05 (m, 1H), 3.97-3.77 (m, 2H), 3.71 (qd, *J* = 10.0, 4.0 Hz, 1H), 3.44 (spt, *J* = 6.8 Hz, 1H), 2.67 (s, 3H), 2.23-2.07 (m, 1H), 2.06-1.96 (m, 1H), 1.85-1.64 (m, 8H), 1.50 (d, *J* = 7.1 Hz, 3H), 1.27-1.05 (m, 10H); LCMS (ESI) m/z: 551.3 (M+23).

### Example 25

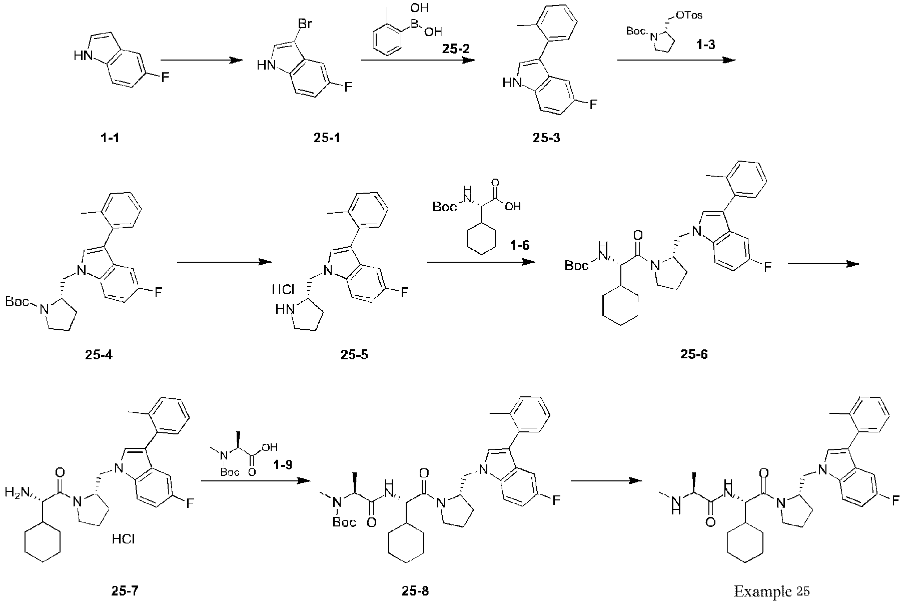

### Step 1:

NBS (276.58 mg, 1.55 mmol, 1.05 equiv.) was added in portions into a solution of Compound 1-1 (0.2 g, 1.48 mmol, 1.0 equiv.) in DMF (2 mL), and the resulting mixture was stirred at 15 °C for 1 hour. TLC (petroleum ether : ethyl acetate = 3 : 1) showed that the starting materials were reacted completely, and LCMS showed that a product was formed. A saturated sodium sulfite solution (2 mL) was added into the reaction solution, and the mixture was extracted with ethyl acetate (2 mL × 3). The combined organic phases were concentrated to obtain Compound 25-1, and the crude product was directly used in the next step. LCMS (ESI) m/z: 211.9 (M-1).

### Step 2:

Under the protection of nitrogen, Compound 25-2 (1.91 g, 14.02 mmol, 2.0 equiv.), potassium phosphate (2.98 g, 14.02 mmol, 2.0 equiv.) and Pd(dppf)Cl₂ (512.80 mg, 700.82 µmol, 0.1 equiv.) were added into a mixed solution of Compound 25-1 (1.5 g, 7.01 mmol, 1.0 equiv.) in tetrahydrofuran (18 mL) and water (3.0 mL), and the resulting mixture was heated to 80 °C and stirred for 16 hours. LCMS showed that the starting materials were reacted completely. The reaction solution was dried over anhydrous sodium sulfate and then filtered, and the filtrate was concentrated under reduced pressure. The resulting residue was purified by silica gel column chromatography (petroleum ether : ethyl acetate = 1 : 0 to 5 : 1) to obtain Compound 25-3. ¹H NMR (400MHz, DMSO-*d*₆) δ 8.07-7.86 (m, 1H), 7.52 (d, *J=* 2.4 Hz, 1H), 7.46-7.40 (m, 1H), 7.37-7.30 (m, 1H), 7.26-7.21 (m, 2H), 7.13-7.05 (m, 2H), 6.99 (dt, *J* = 9.1, 2.3, 1H), 2.29 (s, 3H); LCMS (ESI) m/z: 224.0 (M-1).

### Step 3:

Please refer to the preparation method of Compound 1-4 for the preparation method of Compound 25-4. ¹H NMR (400MHz, CDCl₃) δ 7.43-7.24 (m, 5H), 7.22-7.13 (m, 1H), 7.13-7.07 (m, 1H), 7.05-6.97 (m, 1H), 4.49-4.36 (m, 1H), 4.31-4.24 (m, 1H), 3.50-3.33 (m, 1H), 3.26-3.11 (m, 1H), 2.35 (s, 3H), 1.95-1.85 (m, 1H), 1.83-1.71 (m, 2H), 1.59-1.46 (m, 11H); LCMS (ESI) m/z: 431.3 (M+23).

### Step 4:

Please refer to the preparation method of Compound 1-5 for the preparation method of Compound 25-5. LCMS (ESI) m/z: 309.2 (M+1).

### Step 5:

Please refer to the preparation method of Compound 1-7 for the preparation method of Compound 25-6. LCMS (ESI) m/z: 548.1 (M+1).

### Step 6:

Please refer to the preparation method of Compound 1-8 for the preparation method of Compound 25-7. LCMS (ESI) m/z: 448.2 (M+1).

### Step 7:

Please refer to the preparation method of Compound 1-10 for the preparation method of Compound 25-8. LCMS (ESI) m/z: 633 (M+1).

### Step 8:

Please refer to Example 1 for the preparation method of Example 25. ¹H NMR (400MHz, CD₃OD) δ 7.70 (dd, *J* = 8.9, 4.1 Hz, 1H), 7.31 (dd, *J* = 4.7, 3.2 Hz, 2H), 7.27 (s, 1H), 7.25-7.19 (m, 2H), 7.03-6.94 (m, 2H), 4.62-4.47 (m, 3H), 4.21-4.07 (m, 1H), 3.97-3.88 (m, 1H), 3.86-3.74 (m, 1H), 3.70-3.59 (m, 1H), 2.68 (s, 3H), 2.28 (s, 3H), 2.09-1.57 (m, 11H), 1.52 (d, *J* = 6.9 Hz, 3H), 1.39-1.02 (m, 6H); LCMS (ESI) m/z: 533.2 (M+1).

### Example 26

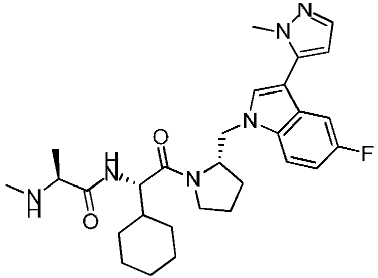

Please refer to Example 25 for the preparation method of Example 26. ¹H NMR (400MHz, CD₃OD) δ 8.23-8.14 (m, 1H), 8.03-7.93 (m, 1H), 7.88 (dd, *J* = 8.9, 4.3 Hz, 1H), 7.39 (br d, *J* = 9.4 Hz, 1H), 7.12 (dt, *J* = 9.1, 2.4 Hz, 1H), 6.94-6.85 (m, 1H), 4.65-4.56 (m, 2H), 4.48 (d, *J* = 7.5 Hz, 1H), 4.25-4.08 (m, 4H), 4.00-3.91 (m, 1H), 3.90-3.80 (m, 1H), 3.79-3.68 (m, 1H), 2.67 (s, 3H), 2.30-2.13 (m, 1H), 2.05-1.95 (m, 1H), 1.90-1.60 (m, 8H), 1.51 (d, *J* = 7.0 Hz, 3H), 1.38-1.01 (m, 6H); LCMS (ESI) m/z: 523.3 (M+1).

### Example 27

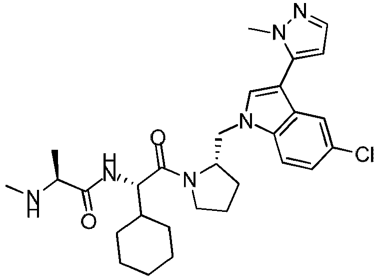

Please refer to Example 25 for the preparation method of Example 27. ¹H NMR (400 MHz, DMSO-*d*₆) δ 7.83-7.95 (m, 2H), 7.82 (s, 1H), 7.53-7.59 (m, 1H), 7.47-7.53 (m, 1H), 7.29 (dd, *J* = 8.78, 2.01 Hz, 1H), 6.48 (d, *J* = 1.76 Hz, 1H), 4.34-4.53 (m, 3H), 4.10 (dd, *J* = 13.05, 8.03 Hz, 1H), 3.86-3.89 (m, 3H), 3.61 (br d, *J* = 8.03 Hz, 2H), 2.97 (q, *J* = 6.69 Hz, 1H), 2.17 (s, 3H), 1.98-2.07 (m, 1H), 1.78-1.92 (m, 2H), 1.51-1.77 (m, 9H), 1.14 (br s, 1H), 1.09 (d, *J* = 6.78 Hz, 4H), 0.89-1.04 (m, 2H); LCMS (ESI) m/z: 539.3 (M+1).

### Example 28

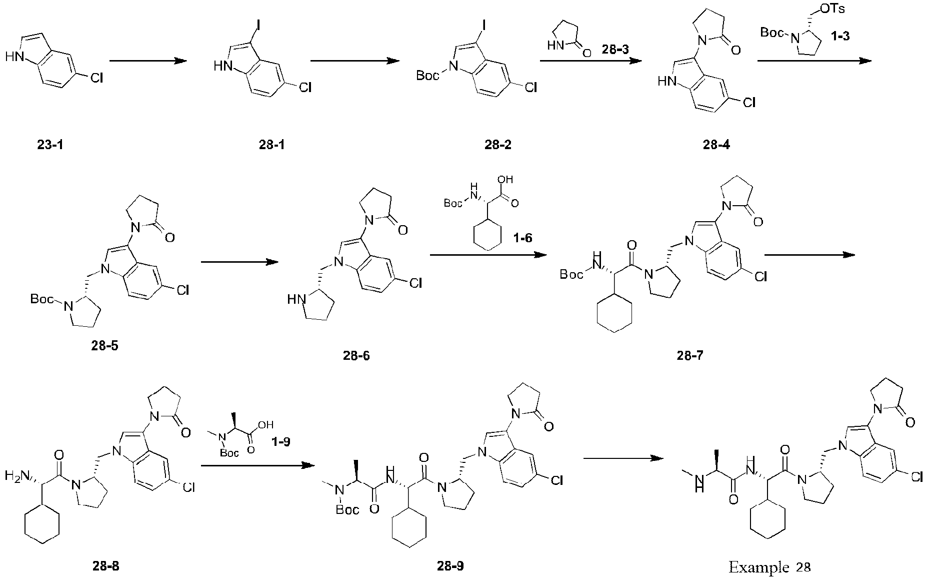

### Step 1:

A solution of iodine (1.67 g, 6.60 mmol, 1.0 equiv.) in DMF (20 mL) and potassium hydroxide (0.92 g, 16.49 mmol, 2.5 equiv.) were added into a solution of Compound 23-1 (1.0 g, 6.60 mmol, 1.0 equiv.) in DMF (20 mL) at 20 °C, and the resulting mixture was stirred and reacted at 20 °C for 1 hour. TLC (petroleum ether : ethyl acetate = 3 : 1) detection showed that the starting materials were reacted completely. The reaction solution was slowly poured into a saturated aqueous sodium sulfite solution (100 mL), and then the mixture was extracted with ethyl acetate (100 mL). The combined organic phases were washed with saturated saline (100 mL) and then concentrated to obtain Compound 28-1. The crude product was used directly in the next step. LCMS (ESI) m/z: 277.9 (M+1).

### Step 2:

Boc₂O (1.42 g, 6.49 mmol, 1.49 mL, 1.2 equiv.), TEA (1.64 g, 16.22 mmol, 1 equiv.) and DMAP (66 mg, 0.54 mmol, 0.1 equiv.) were added into a solution of Compound 28-1 (1.5 g, 5.41 mmol, 1.0 equiv.) in dichloromethane (50 mL). The resulting mixture was stirred and reacted at 20 °C for 10 hours. TLC (petroleum ether : ethyl acetate = 10 : 1) detection showed that the starting materials were reacted completely. The reaction mixture was concentrated, and the resulting residue was purified by silica gel column chromatography (petroleum ether : ethyl acetate = 20 : 1 to 10 : 1) to obtain Compound 28-2. 1H NMR (400MHz, DMSO-d6) δ 8.01 (d, *J =* 8.8 Hz, 1H), 7.91 (s, 1H), 7.39 (dd, *J =* 8.8, 2.0 Hz, 1H), 7.30 (d, *J =* 1.8 Hz, 1H), 1.61 (s, 9H); LCMS (ESI) m/z: 378.0(M+1).

### Step 3:

Under the protection of nitrogen, Compound 28-3 (1.01 g, 11.92 mmol, 0.91 mL, 3.0 equiv.), cuprous iodide (0.37 mg, 1.99 mmol, 0.5 equiv.), cesium carbonate (3.88 g, 11.92 mmol, 3.0 equiv.), and *N*,*N*-dimethylethylenediamine (0.35 g, 3.97 mmol, 1.0 equiv.) were added into a solution of Compound 28-2 (1.5 g, 3.97 mmol, 1.0 equiv.) in dioxane (40 mL). The reaction solution was heated to 80 °C and reacted for 2.0 hours. LCMS showed that the starting materials were reacted completely. The reaction solution was cooled to 20 °C and filtered. The filter cake was washed with ethyl acetate (50 mL), and the filtrate was concentrated. The resulting residue was purified by silica gel column chromatography (petroleum ether : ethyl acetate = 5 : 1 to 1 : 1) to obtain Compound 28-4. ¹H NMR (400MHz, CDCl₃) δ 8.97 (br s, 1H), 7.53 (d, *J=* 1.8 Hz, 1H), 7.19-7.01 (m, 3H), 3.92 (t, *J =* 7.0 Hz, 2H), 2.64 (t, *J=* 8.2 Hz, 2H), 2.32-2.24 (m, 2H); LCMS (ESI) m/z: 234.1 (M+1).

### Step 4:

Please refer to the preparation method of Compound 1-4 for the preparation method of Compound 28-5. ¹H NMR (400MHz, CDCl₃) δ 7.61 (br d, *J=* 6.1 Hz, 1H), 7.48 (br *d, J=* 8.7 Hz, 1H), 7.44-7.38 (m, 1H), 7.32 (br d, *J =* 8.7 Hz, 1H), 7.15 (dd, *J =* 8.8, 1.9 Hz, 1H), 4.22-4.13 (m, 1H), 3.98 (br t, *J =* 7.0 Hz, 2H), 3.45-3.15 (m, 2H), 2.95 (s, 1H), 2.87 (s, 1H), 2.59 (t, *J=* 8.1 Hz, 2H), 2.30-2.16 (m, 2H), 1.93-1.65 (m, 4H), 1.50 (s, 10H); LCMS (ESI) m/z: 418.2 (M+1).

### Step 5:

Please refer to the preparation method of Compound 1-5 for the preparation method of Compound 28-6. LCMS (ESI) m/z: 318.1 (M+1).

### Step 6:

Please refer to the preparation method of Compound 1-7 for the preparation method of Compound 28-7. LCMS (ESI) m/z: 557.3 (M+1).

### Step 7:

Please refer to the preparation method of Compound 1-8 for the preparation method of Compound 28-8. LCMS (ESI) m/z: 457.2 (M+1).

### Step 8:

Please refer to the preparation method of Compound 1-10 for the preparation method of Compound 28-9. LCMS (ESI) m/z: 642.3 (M+1).

### Step 9:

Please refer to Example 1 for the preparation method of Example 28. ¹H NMR (400MHz, DMSO-*d*₆) δ 9.41 (br s, 1H), 9.08-8.72 (m, 2H), 7.84-7.49 (m, 3H), 7.26-7.09 (m, 1H), 4.51-4.23 (m, 3H), 4.10-3.79 (m, 4H), 3.72-3.51 (m, 1H), 2.46 (br s, 6H), 2.14 (br d, *J* = 6.0 Hz, 2H), 1.95 (br s, 1H), 1.90-1.54 (m, 9H), 1.35 (br d, *J* = 6.2 Hz, 3H), 1.29-0.94 (m, 6H); LCMS (ESI) m/z: 542.3 (M+1).

### Example 29

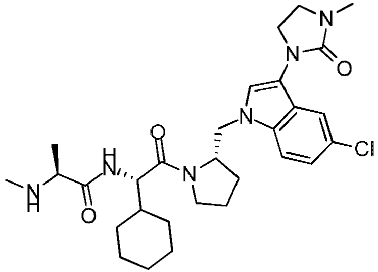

Please refer to Example 28 for the preparation method of Example 29. ¹H NMR (400MHz, CD₃OD) δ 7.67 (d, *J* = 8.8 Hz, 1H), 7.57 (d, *J* = 1.8 Hz, 1H), 7.31 (s, 1H), 7.15 (dd, *J* = 8.7, 1.9 Hz, 1H), 4.59-4.38 (m, 4H), 4.08-3.98 (m, 1H), 3.96-3.90 (m, 1H), 3.88-3.81 (m, 2H), 3.81-3.74 (m, 1H), 3.72-3.64 (m, 1H), 3.61-3.53 (m, 2H), 2.89 (s, 4H), 2.67 (s, 3H), 1.93 (br d, *J=* 9.7 Hz, 2H), 1.88-1.66 (m, 9H), 1.52-1.49 (m, 3H), 1.36-1.08 (m, 6H); LCMS (ESI) m/z: 557.3 (M+1).

### Example 30

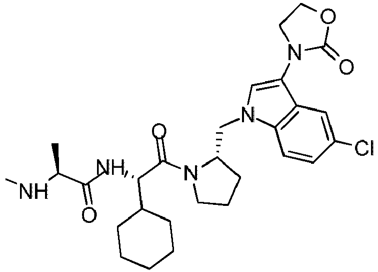

Please refer to Example 28 for the preparation method of Example 30. ¹H NMR (400MHz, CD₃OD) δ 7.70 (*d, J* = 8.8 Hz, 1H), 7.63 (*d, J =* 2.0 Hz, 1H), 7.45 (s, 1H), 7.21-7.14 (m, 1H), 4.60-4.55 (m, 2H), 4.54-4.43 (m, 3H), 4.12 (dt, *J =* 8.1, 3.5 Hz, 2H), 4.07-3.98 (m, 1H), 3.97-3.89 (m, 1H), 3.84-3.75 (m, 1H), 3.73-3.64 (m, 1H), 2.67 (s, 3H), 2.00-1.89 (m, 2H), 1.87-1.76 (m, 6H), 1.71 (br d, *J* = 10.8 Hz, 2H), 1.51 (d, *J* = 7.0 Hz, 3H), 1.36-1.06 (m, 6H); LCMS (ESI) m/z: 544.3 (M+1).

### Example 31

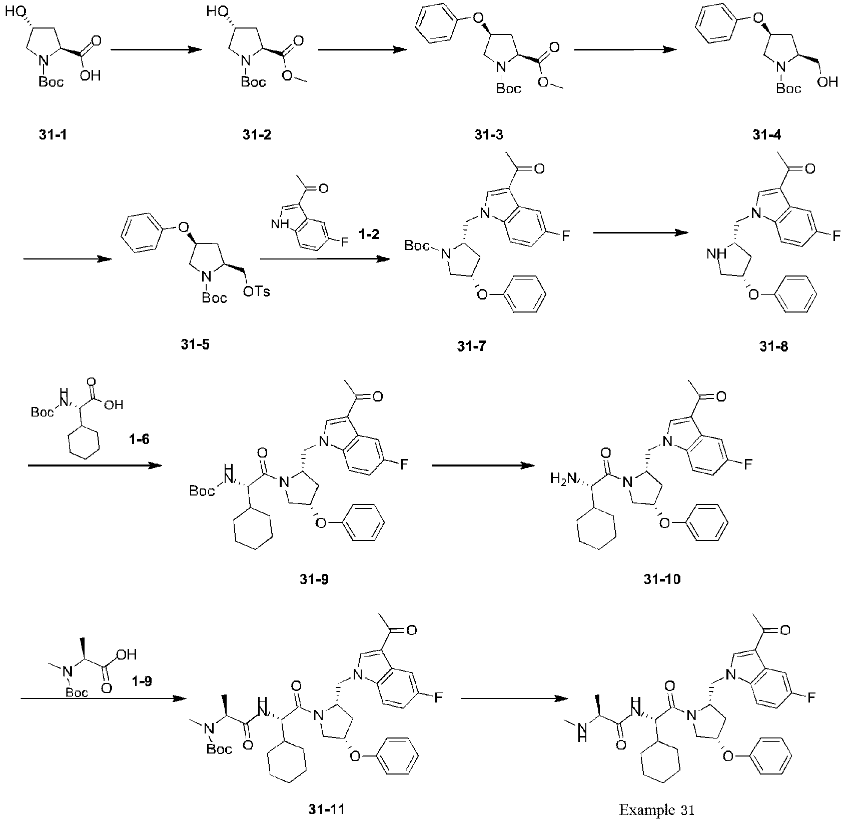

### Step 1:

Potassium carbonate (17.93 g, 129.73 mmol, 3.0 equiv.) and methyl iodide (9.21 g, 64.87 mmol, 4.04 mL, 1.5 equiv.) were added into a solution of Compound 31-1 (10 g, 43.24 mmol, 1.0 equiv.) in DMF (100 mL), and the resulting mixture was reacted at 15 °C for 5 hours. TLC (petroleum ether : ethyl acetate = 1 : 1) showed that the reaction was complete. Water (100 mL) was added into the reaction solution, and the resulting mixture was extracted with ethyl acetate (100 mL × 2). The combined organic phases were washed with saturated saline (100 mL × 3) and then concentrated to obtain Compound 31-2. The crude product was used directly in the next step.

### Step 2:

Under the protection of nitrogen, phenol (3.8 g, 40.36 mmol, 3.55 mL, 1.1 equiv.), triphenylphosphine (10.59 g, 40.36 mmol, 1.1 equiv.) and DIAD (8.16 g, 40.36 mmol, 7.85 mL, 1.1 equiv.) were added into a solution of Compound 31-2 (9.0 g, 36.69 mmol, 1.0 equiv.) in tetrahydrofuran (150 mL). The resulting mixture was stirred at 15 °C for 12 hours. LCMS showed that the reaction was complete. The reaction solution was concentrated, water (100 mL) and ethyl acetate (200 mL) were added into the resulting residue, and the resulting mixture was subjected to liquid-liquid separation. The organic phase was washed with saturated saline (100 mL × 2) and then concentrated to obtain Compound 31-3. The crude product was used directly in the next step. LCMS (ESI) m/z: 322.2 (M+1).

### Step 3:

Lithium aluminum hydride (1.59 g, 42.01 mmol, 1.5 equiv.) was added into a solution of Compound 31-3 (9 g, 28.01 mmol, 1.0 equiv.) in tetrahydrofuran (100 mL) at 0 °C, and the resulting mixture was reacted at 15 °C for 2 hours. LCMS showed that the reaction was complete. To the reaction solution, water (3 mL), a 30% sodium hydroxide solution (6 mL) and water (3 mL) were successively added dropwise to quench the reaction. The resulting mixture was filtered, the filter cake was washed with ethyl acetate (100 mL), and the filtrate was concentrated. The resulting residue was purified by silica gel column chromatography (petroleum ether : ethyl acetate = 10 : to 2 : 1) to obtain Compound 31-4. LCMS (ESI) m/z: 316.2 (M+23).

### Step 4:

Pyridine (4.04 g, 51.13 mmol, 4.13 mL, 3.0 equiv.) and *p*-toluenesulfonyl chloride (6.50 g, 34.09 mmol, 2.0 equiv.) were added into a solution of Compound 31-4 (5 g, 17.04 mmol, 1.0 equiv.) in dichloromethane (150 mL) at 0 °C, and the resulting mixture was stirred at 15 °C for 10 hours. LCMS showed that the reaction was complete. The reaction solution was concentrated, and the resulting residue was purified by silica gel column chromatography (petroleum ether : ethyl acetate = 20 : 1 to 3 : 1) to obtain Compound 31-5. LCMS (ESI) m/z: 470.2 (M+23).

### Step 5:

Please refer to the preparation method of Compound 1-4 for the preparation method of Compound 31-7. LCMS (ESI) m/z: 453.1 (M+1).

### Step 6:

Please refer to the preparation method of Compound 1-5 for the preparation method of Compound 31-8.

### Step 7:

Please refer to the preparation method of Compound 1-7 for the preparation method of Compound 31-9. LCMS (ESI) m/z: 592.1 (M+1).

### Step 8:

Please refer to the preparation method of Compound 1-8 for the preparation method of Compound 31-10.

### Step 9:

Please refer to the preparation method of Compound 1-10 for the preparation method of Compound 31-11. LCMS (ESI) m/z: 677.2 (M+1).

### Step 10:

Please refer to Example 1 for the preparation method of Example 31. ¹H NMR (400MHz, DMSO-*d*₆) δ 9.60-9.34 (m, 1H), 8.98-8.83 (m, 1H), 8.80 *(d, J* = 7.8 Hz, 1H), 7.98 (s, 1H), 7.83 (dd, *J* = 9.8, 2.6 Hz, 1H), 7.73 (dd, *J=* 9.0, 4.5 Hz, 1H), 7.44-7.34 (m, 2H), 7.15-7.08 (m, 3H), 7.07-7.01 (m, 1H), 5.22 (br s, 1H), 4.73-4.56 (m, 2H), 4.37 (t, *J=* 7.6 Hz, 1H), 4.33-4.22 (m, 1H), 4.10 (dd, *J =* 11.8, 4.6 Hz, 1H), 3.93-3.83 (m, 2H), 2.45 (br t, *J =* 5.2 Hz, 4H), 2.31 (s, 3H), 2.21-2.09 (m, 1H), 2.00 (br d, *J =* 14.2 Hz, 1H), 1.71-1.55 (m, 6H), 1.35 (d, *J =* 6.8 Hz, 3H), 1.22-0.92 (m, 6H); LCMS (ESI) m/z: 577.2 (M+1).

### Experimental Example I:

### In-vivo drug efficacy study 1

To evaluate the tumor immunotherapeutic effect of test drugs in the mouse triple-negative breast cancer EMT6 cell line (EMT6 is from ATCC, cell No.: CRL-2755) transplanted BALB/c mouse model.

### Experimental Operation:

BALB/c mice, female, 6-7 weeks old, weighing about 19.1-22.6 g, were housed in separated ventilated boxes with constant temperature and humidity. The feeding room was under the following conditions: temperature: 20-26 °C, humidity: 40-70%, ventilation: 10-20 times/h, and light/dark cycle: 12 h/12 h. The Co-60 radiation-sterilized complete pellet feed for mice was supplied continuously and can be taken freely without limitation, and the drinking tap water (used after autoclaving) was supplied continuously by the water bottle and can be taken freely. A total of 144 mice purchased from Shanghai Lingchang Biotechnology Co., Ltd. were used for the study. The BALB/c mice were inoculated with EMT6 cells subcutaneously to establish murine breast cancer models with subcutaneously-transplanted tumors. The mice were administered when the mean tumor volume reached about 97.3 mm³. The test compounds were administered orally once every 3 days at a dose of 50 mg/kg. PD1 mAb (from BioXcell, cat No.: BP0146, batch No.: 695318A1B, clone No.: RMP1-14) was administered intraperitoneally once every 3 days at a dose of 10 mg/kg. Tumor volume was measured every 3 days with a two-dimensional caliper, and the volume was measured in mm³ and calculated according to the following formula: V = 0.5 a × b², where a and b are the long and short diameters, respectively, of the tumor. Anti-tumor efficacy was determined by dividing the mean tumor increase volume of compound-treated animals by the mean tumor increase volume of untreated animals.

**Results of Experiment:** See Table 1.

**Table 1**

| **Groups** | **Examples** | **Dosage** | **Tumor volume (mm³)** | | | |
|---|---|---|---|---|---|---|
| | | | **Day 0** | **Day 7** | **Day 14** | **Day 25** |
| Group 1 | Blank control | 0 mg/kg | 97.3 | 503.7 | 1049.2 | 2556.9 |
| Group 2 | Anti-PDl | 10 mg/kg | 97.3 | 423.5 | 595.9 | 588.5 |
| Group 3 | Example 2 | 50 mg/kg | 97.3 | 355.5 | 1097.7 | 2225.8 |
| Group 4 | Example 2 | 50 mg/kg | 97.3 | 332.4 | 174.2 | 31.5 |
| | Anti-PDl | 10 mg/kg | | | | |

### Experimental Example II: In vivo efficacy study 2

To evaluate the tumor immunotherapeutic effect of test drugs in the mouse colon cancer MC38 cell line (from Obio Technology (Shanghai) Co., Ltd.) transplanted BALB/c mouse model.

### Experimental operation:

BALB/c mice, female, 6-7 weeks old, weighing about 18-22 g, were kept in a special pathogen-free environment and in separated ventilated cages (3-5 mice per cage). All cages, bedding and water were disinfected prior to use. All animals had free access to standard certified commercial laboratory diets. A total of 48 mice purchased from Shanghai Slac Laboratory Animal Co., Ltd. were enrolled. Each mouse was implanted subcutaneously in the right flank with mouse colon cancer MC38 cells (2×10⁵ cells in 0.1 mL of phosphate buffered saline) for tumor growth. The mice were administered when the mean tumor volume reached about 70 mm³. Test compounds were administered orally twice a week (morning and afternoon) at a dose of 50 mg/kg; PD-1 antibody (from BioXcell, cat. No.: BP0146, batch No.: 640517M2B) was administered intravenously once a week at a dose of 10 mg/kg. Tumor volume was measured every 3 weeks with a two-dimensional caliper, and the volume was measured in mm³ and calculated according to the following formula: V = 0.5 a × b², where a and b are the long and short diameters, respectively, of the tumor. Anti-tumor efficacy was determined by dividing the mean tumor increase volume of compound-treated animals by the mean tumor increase volume of untreated animals.

Results of Experiment: See Table 2.

**Table 2**

| **Groups** | **Examples** | **Dosage** | **Tumor volume (mm³)** | | | |
|---|---|---|---|---|---|---|
| | | | **Day 0** | **Day 5** | **Day 12** | **Day 20** |
| Group 1 | Blank control | 0 mg/kg | 76.0 | 240.5 | 564.8 | 1225.9 |
| Group 2 | Anti-PDl | 10 mg/kg | 91.3 | 219.2 | 400.7 | 671.8 |
| Group 3 | Example 2 | 50 mg/kg | 51.6 | 140.7 | 215.5 | 427.1 |
| | Anti-PDl | 10 mg/kg | | | | |

## Claims

1. A combination, comprising a compound represented by formula (I), an isomer thereof or a pharmaceutically acceptable salt thereof, and an immune checkpoint inhibitor, 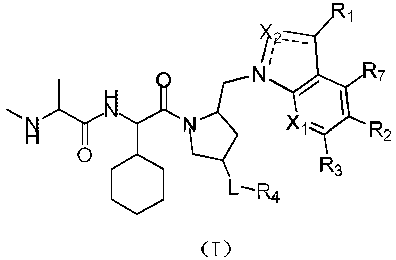 wherein,
X₁ is selected from C(R₅) and N;
X₂ is selected from C(R₆), N, O, and S;
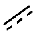 is selected from a single bond and a double bond;
L is selected from a single bond and -O-;
R₁ is selected from -C(=O)NH₂, CN, C₁₋₅ alkyl, C₁₋₅ heteroalkyl, phenyl, 5- to 6-membered heteroaryl, and 5- to 6-membered heterocycloalkyl; the C₁₋₅ alkyl, C₁₋₅ heteroalkyl, phenyl, 5-to 6-membered heteroaryl and 5- to 6-membered heterocycloalkyl are optionally substituted with 1, 2 or 3 R;
R₂ is selected from H, halogen, CN, COOH, -C(=O)NH₂, C₁₋₄ alkyl, and C₁₋₄ heteroalkyl; the C₁₋₄ alkyl and C₁₋₄ heteroalkyl are optionally substituted with 1, 2 or 3 R;
R₃ and R₇ are each independently selected from H, halogen and C₁₋₄ alkyl; the C₁₋₄ alkyl is optionally substituted with 1, 2 or 3 R;
R₄ is selected from H, phenyl, and 5- to 6-membered heteroaryl;
R₅ is selected from H and halogen;
R₆ is selected from H, halogen, C₁₋₄ alkyl, C₁₋₄ heteroalkyl, CN, and COOH; the C₁₋₄ alkyl and C₁₋₄ heteroalkyl are optionally substituted with 1, 2 or 3 R;
R is selected from halogen, OH, CN, CH₃, CH₃CH₂, CH₃CH₂CH₂, CH(CH₃)₂, OCH₃, OCF₃, CHF₂, CH₂F, and NH₂; and
the C₁₋₄ heteroalkyl, C₁₋₅ heteroalkyl, 5- to 6-membered heterocycloalkyl and 5- to 6-membered heteroaryl each contain 1, 2 or 3 heteroatoms or heteroatom radicals independently selected from -NH-, -O-, -S-, N, -C(=O)O-, -C(=O)-, -C(=O)NH-, -C(=S)-, -S(=O)-, -S(=O)₂-, -C(=NH)-, -S(=O)₂NH-, -S(=O)NH-, and -NHC(=O)NH-.

2. The combination according to claim 1, wherein the compound represented by formula (I), an isomer thereof or a pharmaceutically acceptable salt thereof is selected from 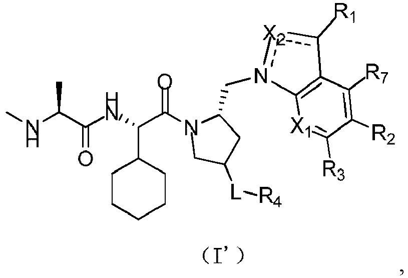 wherein 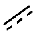 , X₁, X₂, L, R₁, R₂, R₃, R₄ and R₇ are as defined in claim 1.

3. The combination according to claim 1 or 2, wherein X₂ is selected from C(R₆) and N.

4. The combination according to claim 3, wherein X₂ is selected from C(H), C(Cl), C(CH₃), and N.

5. The combination according to claim 1 or 2, wherein R₁ is selected from -C(=O)NH₂, CN, CH₃, CH₃CH₂, C₁₋₅ alkyl-C(=O)-, C₁₋₄ alkyl-C(=O)-, C₁₋₅ alkyl-S(=O)₂-, C₁₋₅ alkyl-N(H)C(=O)-, C₁₋₄ alkyl-N(H)C(=O)-, (C₁₋₂ alkyl)₂-N-C(=O)-, phenyl, 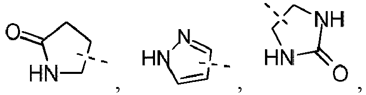 and 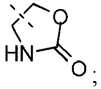 the CH₃, CH₃CH₂, C₁₋₅ alkyl-C(=O)-, C₁₋₄ alkyl-C(=O)-, C₁₋₅ alkyl-S(=O)₂-, C₁₋₅ alkyl-N(H)C(=O)-, C₁₋₄ alkyl-N(H)C(=O)-, (C₁₋₂ alkyl)₂-N-C(=O)-, phenyl, 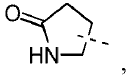 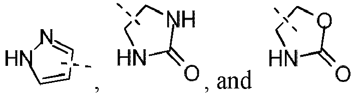 are optionally substituted with 1, 2 or 3 R.

6. The combination according to claim 5, wherein R₁ is selected from

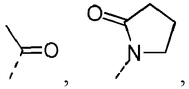

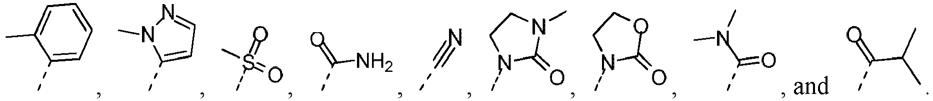

7. The combination according to claim 1 or 2, wherein R₂ is selected from H, halogen, C₁₋₄ alkyl, and C₁₋₄ alkyl-O-; the C₁₋₄ alkyl and C₁₋₄ alkyl-O- are optionally substituted with 1, 2 or 3 halogens.

8. The combination according to claim 7, wherein R₂ is selected from H, F, Cl, Br, CF₃, and OCF₃.

9. The combination according to claim 1 or 2, wherein R₃ and R₇ are each independently selected from H, F, and Cl.

10. The combination according to claim 1 or 2, wherein R₄ is selected from H and

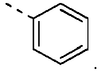

11. The combination according to claim 1 or 2, wherein R₅ is selected from H and Cl.

12. The combination according to claim 1 or 2, wherein R₆ is selected from H, Cl, and CH₃.

13. The combination according to claim 1 or 2, wherein a structural unit 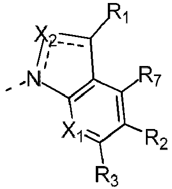 is selected from

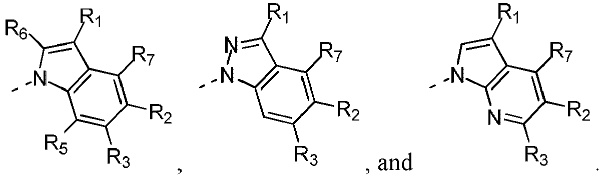

14. The combination according to claim 1 or 2, wherein a structural unit 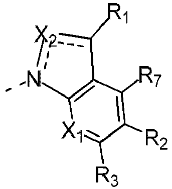 is selected from 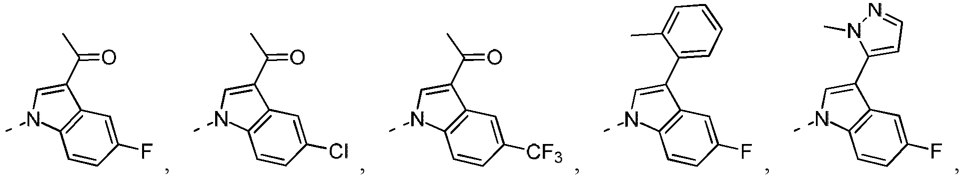 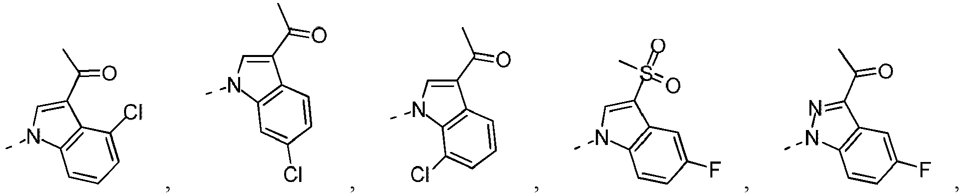 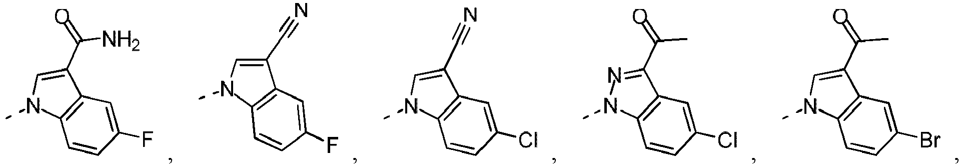 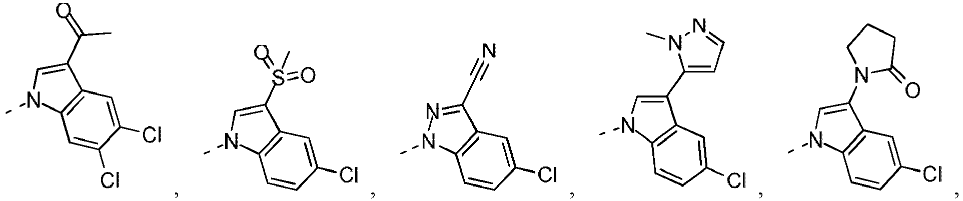 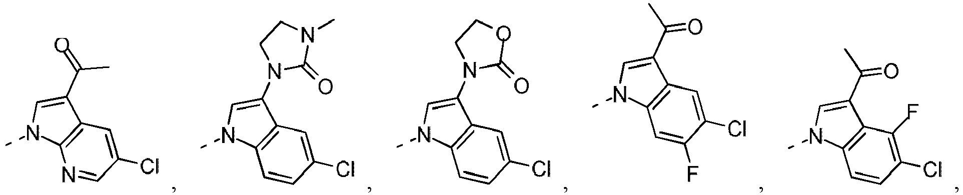 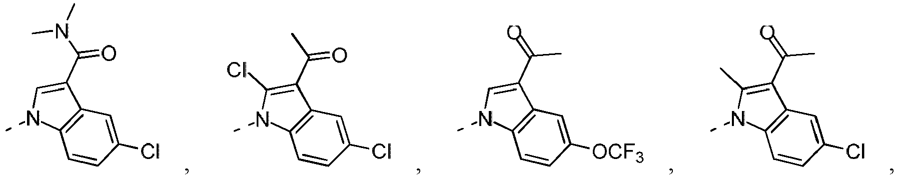 and

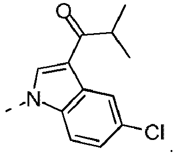

15. The combination according to any one of claims 1 and 7 to 9, wherein the compound, an isomer thereof or a pharmaceutically acceptable salt thereof is selected from 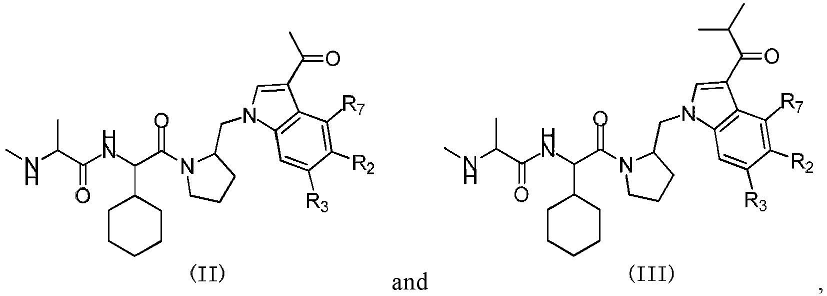 wherein R₂, R₃ and R₇ are as defined in claims 1 to 2 and 7 to 9.

16. The combination according to claim 2 or 15, wherein the compound, an isomer thereof or a pharmaceutically acceptable salt thereof is selected from 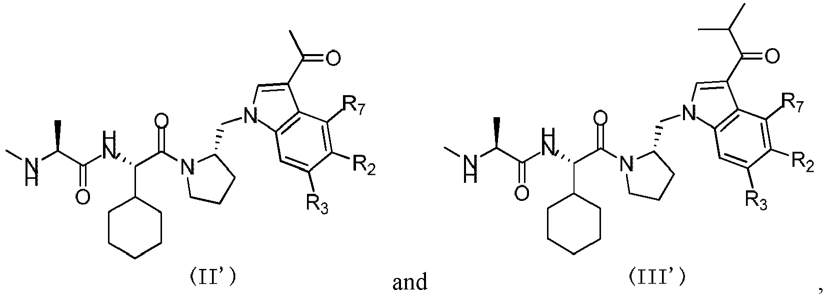 wherein R₂, R₃ and R₇ are as defined in claim 2 or 15.

17. The combination according to claim 1, wherein the compound represented by formula (I), an isomer thereof or a pharmaceutically acceptable salt thereof is selected from

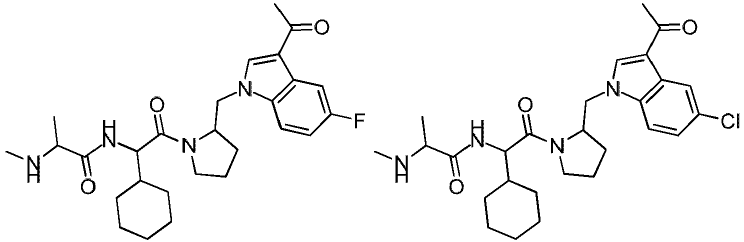

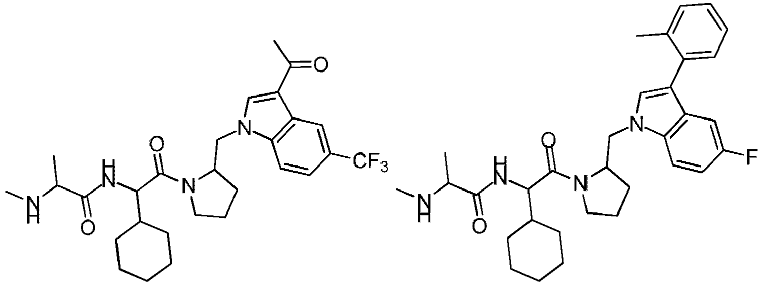

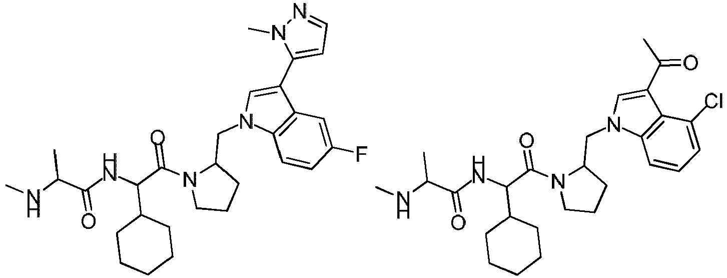

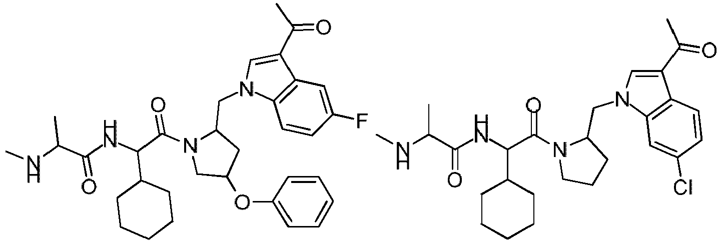

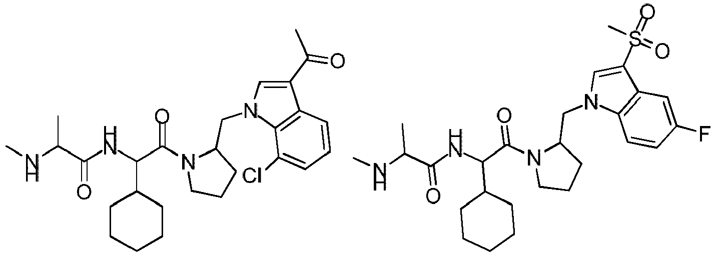

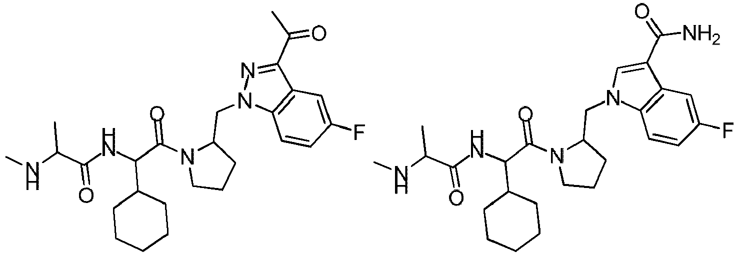

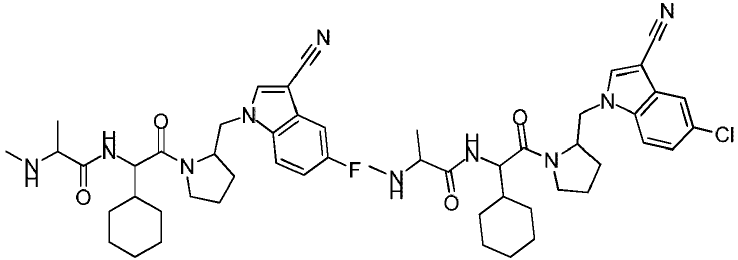

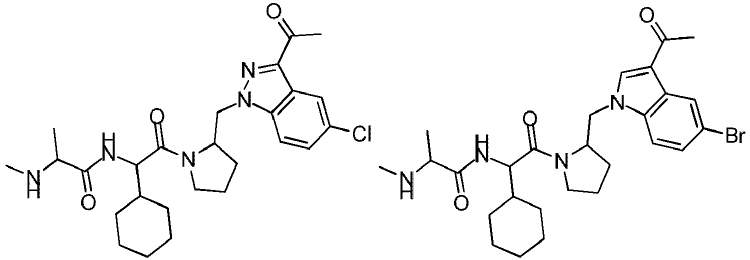

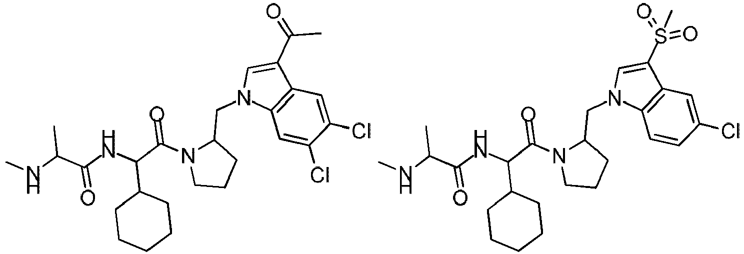

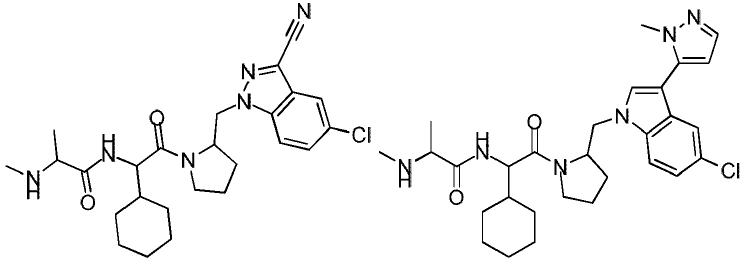

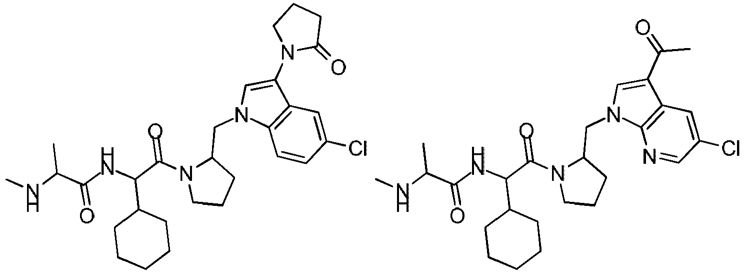

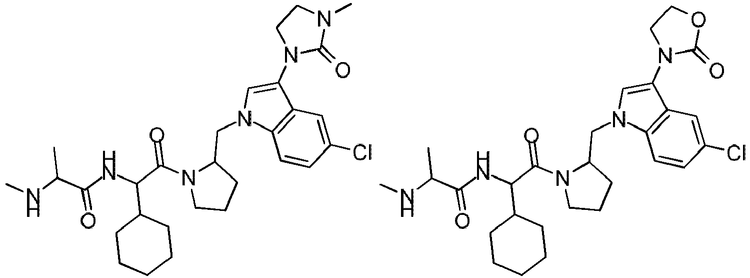

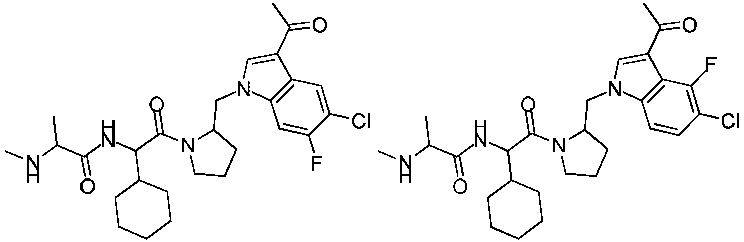

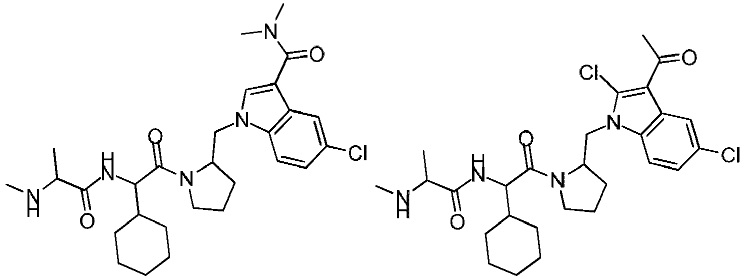

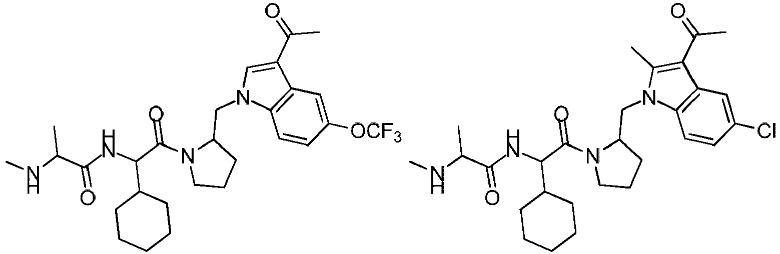

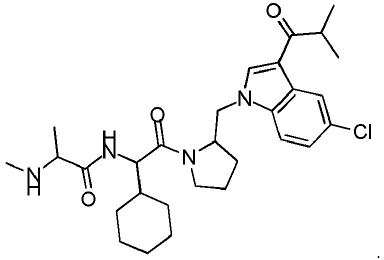

18. The combination according to claim 17, wherein the compound represented by formula (I), an isomer thereof or a pharmaceutically acceptable salt thereof is selected from

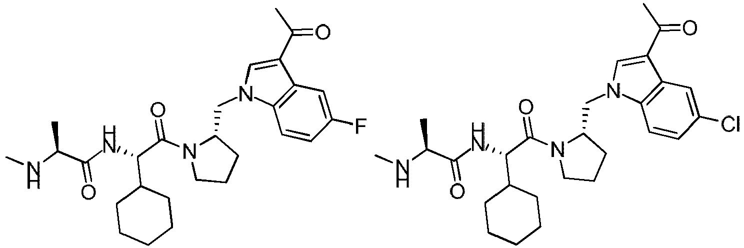

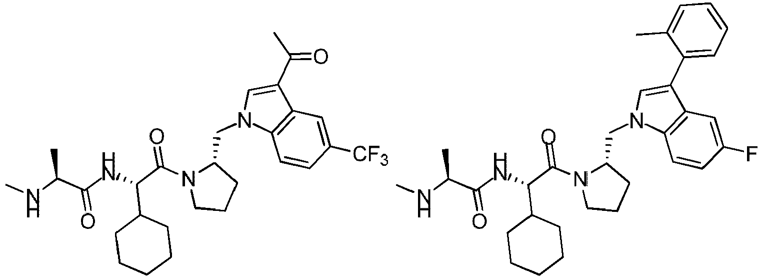

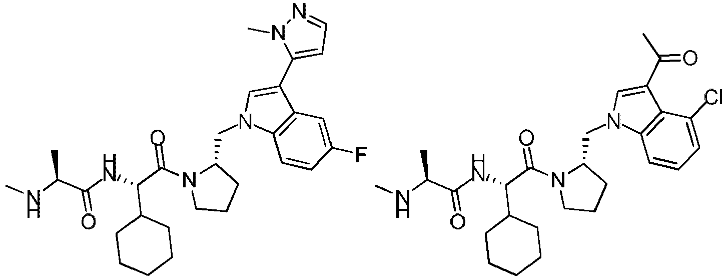

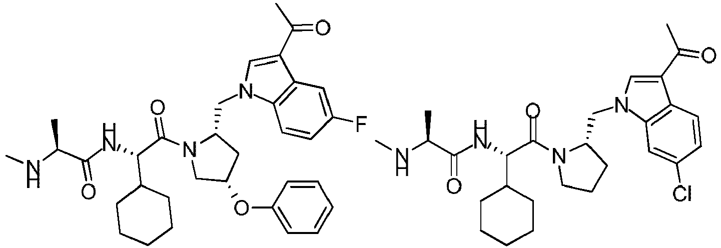

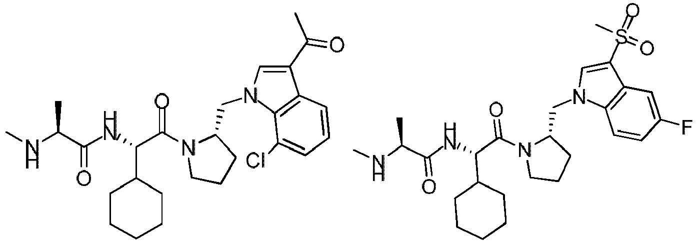

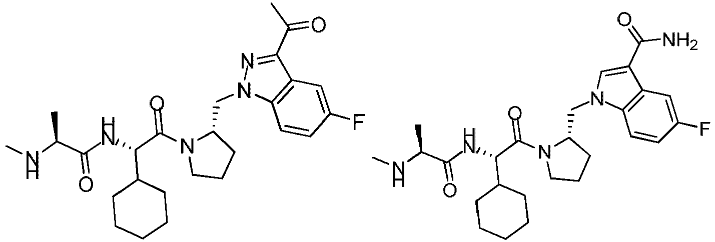

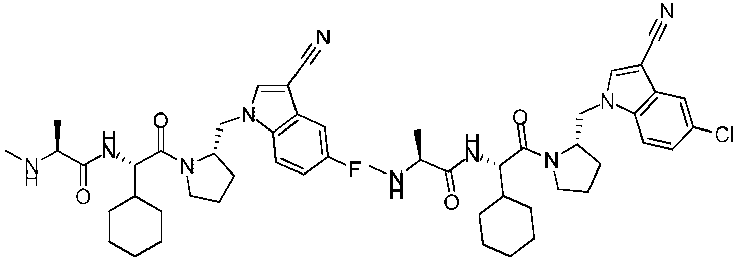

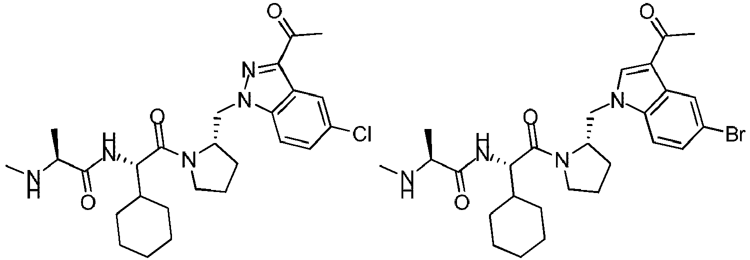

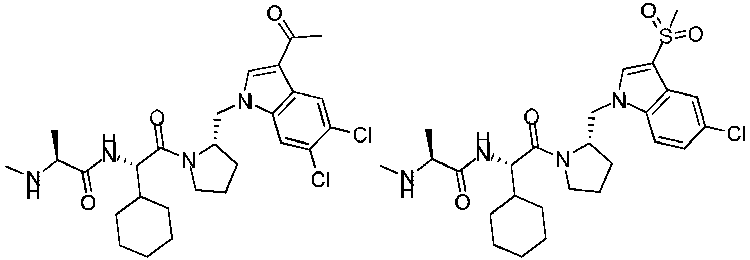

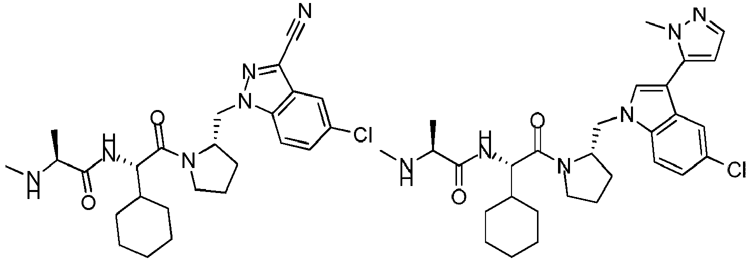

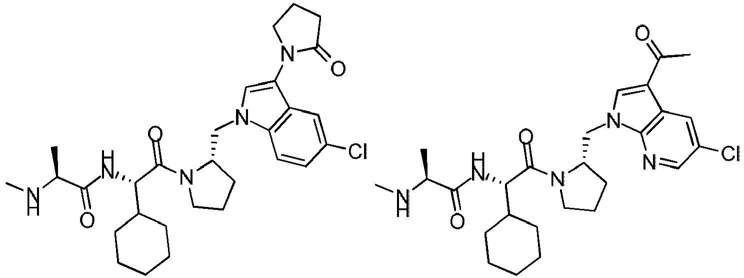

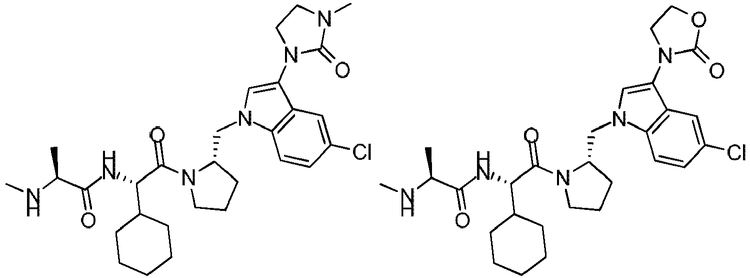

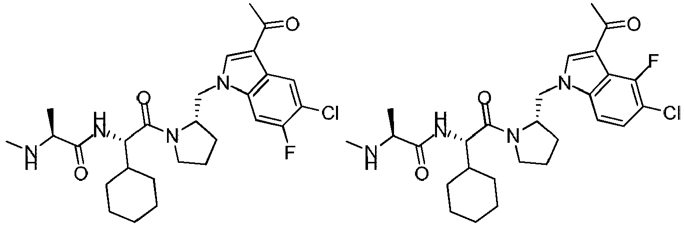

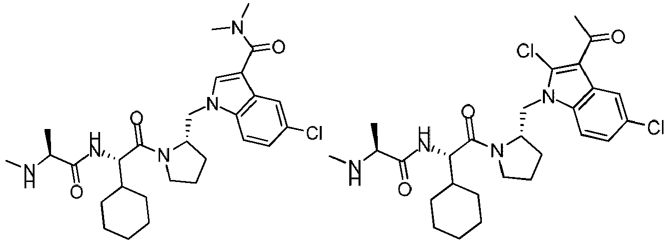

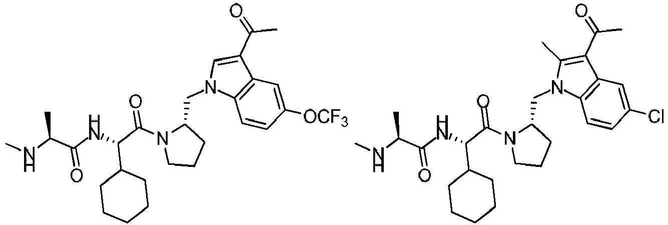

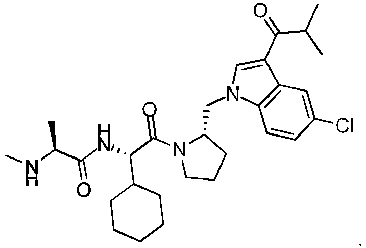

19. The combination according to any one of claims 1 to 18, wherein the immune checkpoint inhibitor is selected from an anti-PD-1 antibody, an anti-PDLl-antibody, and an anti-CTLA-4 antibody.

20. The combination according to any one of claims 1 to 18, wherein the combination is a combination for use in the treatment of cancer.

21. A combined pharmaceutical composition, comprising the combination according to any one of claims 1 to 20 and a pharmaceutically acceptable excipient.

22. A kit, comprising the combined pharmaceutical composition according to claim 21 and instructions for use of a compound represented by formula (I), an isomer thereof or a pharmaceutically acceptable salt thereof in combination with an immune checkpoint inhibitor in the treatment of cancer.

23. Use of the combination according to any one of claims 1 to 18, the combined pharmaceutical composition according to claim 21 or the kit according to claim 22 in the preparation of a cancer treatment drug.
